# EUROPEAN PATENT SPECIFICATION

(11) **EP 2 228 028 B1**
(45) Date of publication and mention of the grant of the patent: **22.04.2015**
(21) Application number: 10168186.4
(22) Date of filing: 27.09.2006
(51) Int. Cl.: A61B 19/00, A61B 17/3205, A61B 17/34, A61F 2/10

(54) **Apparatus for harvesting and implanting follicular units**
Gerät zur Gewinnung und Implantation von follikulären Einheiten
Appareil d'extraction et d'implantation d'unités folliculaires

(30) Priority: 30.09.2005 US 722521 P; 22.12.2005 US 753602 P; 31.01.2006 US 764173 P; 28.04.2006 US 380903; 28.04.2006 US 380907; 28.04.2006 US 380911; 31.05.2006 US 421438; 31.05.2006 US 421443
(43) Date of publication of application: 15.09.2010
(62) Divisional of application: 06804246.4
(73) Proprietor: Restoration Robotics, Inc., San Jose, CA 95134 (US)
(72) Inventor: Bodduluri, Mohan, Palo Alto, California 94036 (US); Gildenberg, Philip L., Houston, Texas 77005 (US); Caddes, Donald E., Menlo Park, California 94025 (US); Hsei, Paul K., Huntington Beach, California 92646 (US)
(74) Representative: Hatzmann, Martin

(56) References cited:
- EP-A1- 1 312 315
- WO-A1-2006/071728
- US-A- 4 160 453
- US-A1- 2002 103 500
- US-A1- 2004 193 203

## Description

### FIELD OF INVENTION

This invention relates generally to an image-guided robotics system for performing precision diagnostic and therapeutic medical procedures.

### BACKGROUND

Hair transplantation procedures are well-known, and typically involve (e.g., in a patient having male pattern baldness) harvesting donor hair grafts from the side and back fringe areas ("donor areas") of the patient's scalp, and implanting the harvested follicular units in a bald, top area ("recipient area"). Historically, the harvested grafts were relatively large (3-5 mm), although more recently, the donor grafts may be single follicular units, which are naturally occurring aggregates of 1-3 (and much less commonly, 4-5) closely spaced hair follicles that are distributed randomly over the surface of the scalp.

In one well-known process, a linear portion of the scalp is removed from a donor area using a scalpel cutting down into the fatty subcutaneous tissue. The strip is dissected (under a microscope) into component follicular units, which are then implanted into a recipient area in respective puncture holes made using a needle. Forceps may be used to grasp and place the individual follicular unit grafts into the needle puncture locations, although other instruments and methods are known for performing this task.

In "Androgenetic Alopecia" (Springer 1996), M. Inaba & Y. Inaba disclose and describe a method for harvesting singular follicular units by positioning a hollow punch needle having a cutting edge and interior lumen with a diameter of 1 mm, which is about equal to the diameter of critical anatomical parts of a follicular unit. The needle punch is axially aligned with an axis of a follicular unit to be extracted and then advanced into the scalp to cut the scalp about the circumference of the selected follicular unit. Thereafter, the follicular units are easily removed, e.g., using forceps, for subsequent implantation into a recipient site with a specially devised insertion needle.

Published U.S. Patent Application 20050203545 (Cole) discloses an instrument for the extraction of individual follicular units that purportedly allows for a more precise penetration depth and angle with respect to the skin surface of the skin of a patient.

Published U.S. Patent Application 20050267506 (Harris) discloses a method and apparatus for the extraction of follicular units by first scoring the outer skin layers with a sharp punch, and then inserting a separate blunt punch into the incision to separate the hair follicular unit from the surrounding tissue and fatty layer.

U.S. Patent No. 6,585,746 (Gildenberg) discloses a hair transplantation system utilizing a robot, including a robotic arm and a hair follicle introducer associated with the robotic arm. A video system is used to produce a three-dimensional virtual image of the patient's scalp, which is used to plan the scalp locations that are to receive hair grafts implanted by the follicle introducer under the control of the robotic arm. However, many improvements both to the provisioning of an automated (e.g., robotic) system and methods of their use for harvesting and implanting hair follicular units, collectively, "transplanting" when referring to the harvesting of a follicular unit from a donor region of a body surface and implanting the harvested unit in a recipient region of the body surface as part of a same procedure.

EP 1 312 315 discloses an apparatus for harvesting and implanting hair follicular units according to the preamble of appended independent claim 1.

### SUMMARY

The invention is defined in appended independent claim 1, preferred embodiments are described in the dependent claims.

In accordance with a general aspect of the inventions disclosed herein, an automated system, such as an image-guided robotics system, is employed for performing precisely controlled harvesting and implantation of hair follicular units. In some embodiments, the automated system includes a moveable arm, a tool mounted on the moveable arm, one or more cameras mounted on the moveable arm, a processor configured to receive and process images acquired by the one or more cameras, and a controller operatively associated with the processor and configured to position the moveable arm based, at least in part, on processed images acquired by the one or more cameras, wherein the moveable arm is positionable such that the tool may be positioned at a desired orientation relative to an adjacent body surface.

By way of non-limiting example, the automated system may be a robotic system, wherein the moveable arm is a robotic arm, and wherein the processor and controller may be configured for positioning the tool by visual-servoing of the robotic arm. In some embodiments, a single camera may be employed, wherein the processor is configured to register a reference coordinate system of the camera with a tool frame reference coordinate system of the robotic arm. For example, the processor may register the camera reference coordinate system with the tool frame reference coordinate system based on images of a fixed calibration target acquired as the robotic arm is moved along one or more axes of the tool frame reference coordinate system. By way of another example, a pair of cameras may be mounted to the robotic arm, wherein the processor is configured to register respective reference coordinate systems of the cameras with each other and with a tool frame reference coordinate system of the robotic arm. Again, the processor may register the respective camera reference coordinate systems with the tool frame reference coordinate system based on images of a fixed calibration target acquired as the robotic arm is moved along one or more axes of the tool frame reference coordinate system. By way of yet another example, the one or more cameras comprises respective first and second pairs of cameras mounted to the robotic arm, the first pair focused to acquire images of a first field of view, and the second pair focused to acquire images of a second field of view substantially narrower than the first field of view. In this embodiment, the processor may be configured to register respective reference coordinate systems of the first and second pairs of cameras with each other and with a tool frame reference coordinate system of the robotic arm. Again, the processor may register the respective camera reference coordinate systems with the tool frame reference coordinate system based on images of a fixed calibration target acquired as the robotic arm is moved along one or more axes of the tool frame reference coordinate system.

In various embodiments, the tool comprises one or both of a follicular unit harvesting tool and a follicular unit implantation tool. In various embodiments, the processor may be configured to identify approximate physical boundaries of a follicular unit in an image acquired by the one or more cameras. For example, the processor may be configured for identifying approximate physical boundaries of a follicular unit captured in an acquired image, including a subcutaneous base region embedded in the body surface and a distal tip region extending away from the body surface, wherein the images include subcutaneous images. In yet another embodiment, an air jet is provided on the moveable arm for directing an air stream at the body surface. In yet another embodiment, a user interface is provided for a user to input instructions to one or both of the processor and controller regarding one or more of a location, position, orientation, and depth of a follicular unit to be implanted.

In accordance with another aspect of the present disclosure, a method for harvesting follicular units from a body surface includes (i) acquiring images of a body surface; (ii) processing the acquired images to identify a follicular unit on the body surface and to determine a relative position and orientation of the identified follicular unit; (iii) using an automated system including a moveable arm to position a harvesting tool mounted on the moveable arm adjacent the identified follicular unit, based at least in part on processed image data, such that a longitudinal axis of the harvesting tool is aligned with a longitudinal axis of the follicular unit; and (iv) harvesting the follicular unit by movement of the harvesting tool relative to the body surface, wherein the images are acquired from one or more cameras mounted on the moveable arm.

In one embodiment, the automated system may be a robotic system, wherein the moveable arm is a robotic arm. In such an embodiment, the images may be acquired from a single camera mounted to the robotic arm, the method further comprising registering a reference coordinate system of the camera with a tool frame reference coordinate system of the robotic arm. For example, the camera reference coordinate system may be registered with the robotic arm tool frame reference coordinate system based on images of a fixed calibration target acquired as the robotic arm is moved along one or more axes of the robotic arm tool frame reference coordinate system. In another such embodiment, the images may be acquired from a pair of cameras mounted to the robotic arm, the method further comprising registering respective reference coordinate systems of the cameras with each other and with a tool frame reference coordinate system of the robotic arm. In still another such embodiment, the images may be acquired using respective first and second pairs of cameras mounted to the robotic arm, the first pair focused to acquire image data of a first field of view, and the second pair focused to acquire image data of a second field of view substantially narrower than the first field of view. In yet a further such embodiment, the method may further comprise identifying the approximate physical boundaries of the identified follicular unit, including (by way of non-limiting example) identifying a subcutaneous base region embedded in the body surface and a distal tip region extending away from the body surface.

In accordance with another aspect of the present disclosure, a method for implanting follicular units in a body surface includes (i) acquiring and processing images of a body surface to identify an implantation site; (ii) using an automated system including a moveable arm to position an implantation tool mounted on the moveable arm to a location adjacent the implantation site; and (iii) implanting a follicular unit in the body surface by movement of the implantation tool relative to the body surface, wherein the images are acquired from one or more cameras mounted on the moveable arm.

By way of non-limiting example, the automated system may be a robotic system, and the moveable arm may be a robotic arm, wherein the implantation tool may be positioned at the implantation site by visual-servoing of the robotic arm. In various embodiments, the follicular unit may be carried in the implantation tool prior to implantation. In various embodiments, the follicular unit is implanted at a desired position and orientation relative to the body surface, and may also be implanted at a desired depth in the body surface. In some embodiments, the method may further include directing an air stream at the implantation site prior to or contemporaneous with implanting the follicular unit, e.g., to clear away the neighboring hairs and/or blood from adjacent implants. In some embodiments, the method may also include inputting through a user interface of the automated system instructions regarding one or more of a location, position, orientation, and depth of a follicular unit to be implanted.

In accordance with yet another aspect of the present disclosure, a method for transplanting follicular units includes (i) acquiring and processing images of a first area of a body surface to identify and determine a relative position and orientation of a follicular unit to be harvested; (ii) using an automated system including a moveable arm to position a harvesting tool mounted on a moveable arm adjacent the identified follicular unit, such that a longitudinal axis of the harvesting tool is aligned with a longitudinal axis of the follicular unit; (iii) harvesting the follicular unit by movement of the harvesting tool relative to the body surface; (iv) acquiring and processing the images of a second area of the body surface to identify an implantation site; (v) using the automated system to position an implantation tool mounted on the moveable arm adjacent the implantation site, and (vi) implanting the follicular unit by movement of the implantation tool relative to the body surface, wherein the respective images are acquired from one or more cameras mounted on the moveable arm.

In accordance with still another aspect of the invention, a multi-part tool assembly is provided for the harvesting and implantation of hair follicular units in a body surface, such as a human scalp. In one embodiment, the tool assembly comprises a pair of coaxially disposed cannulas positioned in a reciprocating relationship, including an outer "implanting" cannula having an interior lumen and an open, tissue-piercing distal end, and an inner "harvesting" cannula positioned in the implanting cannula lumen. The harvesting cannula has an open, tissue-coring distal end, and an interior lumen sized to frictionally engage and retain a follicular unit. The tool assembly may be hand-held and positioned. In the alternative, the tool assembly may be attached to, and positioned by, a moveable arm of an automated system, e.g., a robotic arm system. Movement of one or both of the harvesting and implanting cannulas relative to each other and/or to the remainder of the tool assembly (whether hand-held or carried by an automated positioning system) may be provided by a number of different mechanical, electro-mechanical, pneumatic, hydraulic, magnetic, and other known systems and mechanisms for effecting controlled movement of the respective cannulas. While the implanting and harvesting cannulas are preferably axially aligned, other embodiments are possible.

For harvesting, a longitudinal axis of the harvesting cannula is axially aligned with a longitudinal axis of a selected follicular unit to be harvested. Depending on the embodiment, positioning of the harvesting cannula relative to the selected follicular unit may be manual or fully automated. In one embodiment, an image-guided robotic system including a robotic arm is used to position and align the respective harvesting cannula and follicular unit. The harvesting cannula is advanced over the follicular unit, with its distal coring end penetrating the body surface into the subcutaneous fatty layer surrounding and underlying the follicular unit. The harvesting cannula is then withdrawn from the body surface to thereby extract the follicular unit, which is carried in the harvesting cannula lumen.

Movement of the harvesting cannula relative to the body surface may be manual, semi-automated, or completely automated. The harvesting cannula may be fixed or independently moveable relative to the remainder of the tool assembly, whether the tool assembly is hand-held or attached to a moveable arm. In embodiments in which the tool assembly is carried on an automated (e.g., robotic) arm, movement of the harvesting cannula relative to the body surface may be performed by movement of the arm relative to the body surface, movement of the harvesting cannula relative to the automated arm, or a combination of each. Similarly, in hand-held embodiments, movement of the harvesting cannula relative to the body surface may be performed by movement of the operator's arm relative to the body surface, movement of the harvesting cannula relative to the tool assembly, or a combination of each. In some embodiments, the harvesting cannula is rotated about its longitudinal axis as it penetrates the body surface to enhance its tissue-coring effectiveness. In some embodiments, the wall of the harvesting cannula lumen may be textured in order to facilitate grasping and extracting the follicular unit. In some embodiments, a vacuum source may be selectively placed in communication with the harvesting cannula lumen to apply a proximally directed "pulling" force to facilitate grasping and extracting the follicular units. These features may also be helpful in retaining the follicular unit in the harvesting cannula lumen after it is harvested.

For implantation, the tool assembly is repositioned (whether manually or by using an automated system) to a selected implantation site in a recipient area on the body surface. A longitudinal axis of the implanting cannula may be aligned with a desired orientation of the follicular unit, when implanted. Again, this alignment may be performed manually or by an automated system, e.g., by using an image-guided robotic system in one embodiment. The tissue-piercing distal end of the implanting cannula is advanced into the body surface, creating a subcutaneous implantation cavity of an appropriate depth and size for receiving a follicular unit being implanted. This "puncture motion" by the implanting cannula is preferably very rapid in order to minimize trauma to the tissue surface in the implantation cavity, e.g., akin to the motion of a spring-loaded finger pricking device used for obtaining small amounts of blood for testing.

In one embodiment, a follicular unit is moved axially from the harvesting cannula lumen (where it has remained undisturbed since it was harvested) into the distal end portion of the implanting cannula lumen by an obturator (plunger) disposed in the harvesting cannula lumen. This repositioning of the follicular unit may take place before, during, or after the implanting cannula punctures the body surface. The obturator thereafter maintains the relative position of the follicular unit in the implantation cavity as the implanting cannula is withdrawn from the body surface by translational movement relative to the obturator. In others embodiment, the follicular unit is deposited directly from the harvesting cannula lumen into the implantation cavity, e.g., by the obturator, or by applying a distally directed "pushing" force using a source of pressured air placed in communication with the harvesting cannula lumen.

In accordance with yet another aspect of the present disclosure, a method of transplanting a hair follicular unit using a multi-part tool assembly includes (i) aligning a longitudinal axis of an inner ("harvesting") cannula with a longitudinal axis of a selected follicular unit to be harvested from a donor area of a body surface; (ii) advancing the harvesting cannula relative to the body surface so that an open, tissue coring distal end of the harvesting cannula penetrates the body surface surrounding the selected follicular unit to a depth sufficient to substantially encapsulate the follicular unit; (iii) withdrawing the harvesting cannula from the body surface with the follicular unit engaged by and retained in an interior lumen thereof; (iv) advancing an outer ("implanting") cannula over the coaxially disposed harvesting cannula so that a tissue piercing distal end of the implanting cannula punctures a recipient area of the body surface and forms an implantation cavity therein; and (v) displacing the follicular unit from the harvesting cannula lumen into the implantation cavity.

### BRIEF DESCRIPTION OF THE DRAWINGS

The invention is illustrated by way of example and not limitation in the figures of the accompanying drawings, in which like references indicate similar elements, and in which:
Fig. 1 is a perspective view of a robotic arm system used for positioning and orienting a pair of coaxially-disposed cannulas extending from a distal opening of a tool assembly housing carried by the robotic arm and used for harvesting and implanting human hair follicular units.
Fig. 2 is a close-up of the distal portion of the tool assembly housing shown in Fig. 1.
Fig. 2A is a close-up the distal end portion of an alternate embodiment of the robotic arm system of Fig. 1, in which first and second stereo camera pairs are secured to the robotic arm and used to capture image data from multiple fields-of-view for guiding movement of the robotic arm and attached tool assembly.
Fig. 3 is a perspective view of a multi-part tool for use in the tool assembly in the system of Fig. 1.
Fig. 4 is lengthwise sectional view of the multi-part tool of Fig. 3.
Fig. 5 is a perspective view of a motor drive assembly for operatively coupling with the multi-part-part tool of Fig. 3 in the tool assembly of the system of Fig. 1.
Figs. 6A and 6B are simplified, partially cut-away views of alternative implantation procedures carried out using the three-part tool of Fig. 3.
Fig. 7 is a partial-schematic, partial perspective view of one embodiment of the tool assembly of the robotic system in Fig. 1. Fig. 8 is a partially cut-away sectional view of a holding unit located within a motor drive assembly in the tool assembly of Fig. 7.
Fig. 9A is a lengthwise sectional view of a multi-part tool for use in the tool assembly in the system of Fig. 7.
Figs. 9B-9D illustrate variations of a distal end of a follicular unit harvesting cannula needle of the tool assembly of Fig. 9A.
Fig. 10 illustrates the multi-part tool of Fig. 9A operatively engaged with the holding unit of Fig. 8.

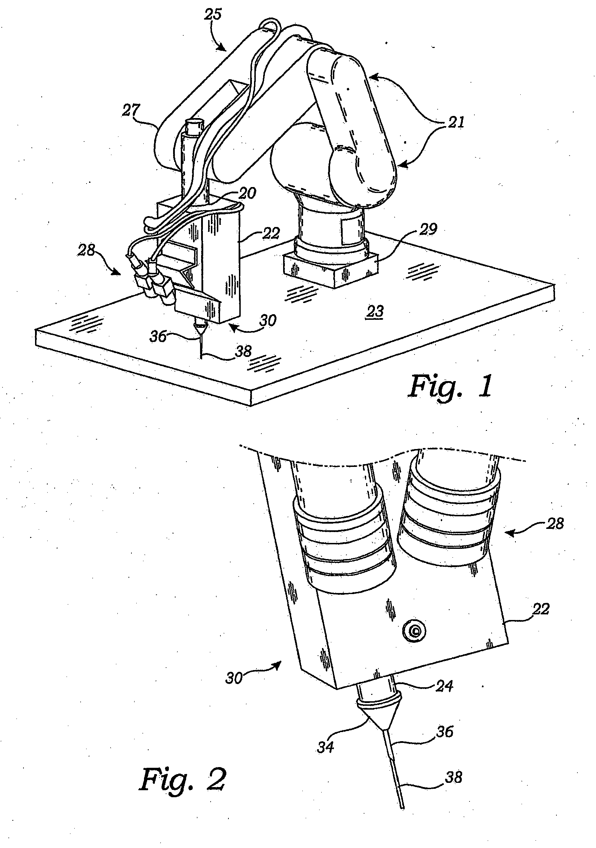

Figs. 1 IA-D illustrate a process for implanting a follicular unit, in accordance with some embodiments.

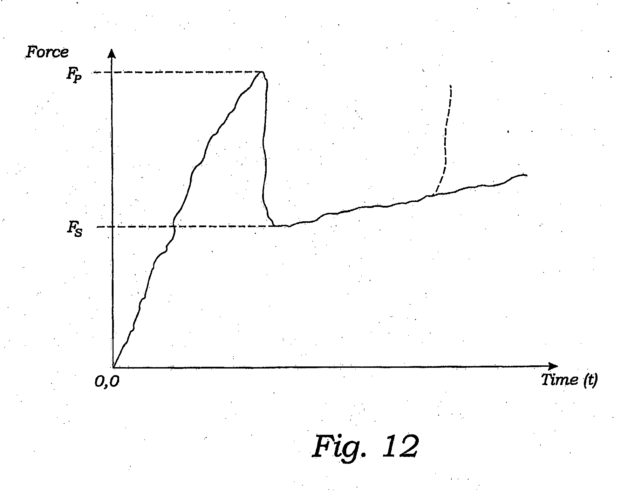

Fig. 12 illustrates a force diagram representing a force experienced by a harvesting cannula, in accordance with some embodiments.

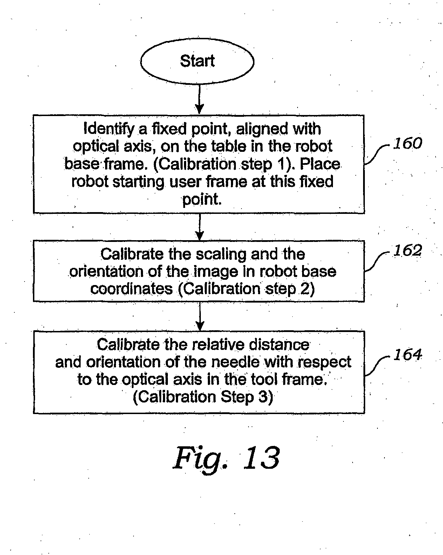

Fig. 13 is a flow diagram of a procedure for calibrating an optical axis and associated camera reference frame of a single camera with a tool frame established at the distal (working) end of the robotic arm to which the camera is attached.

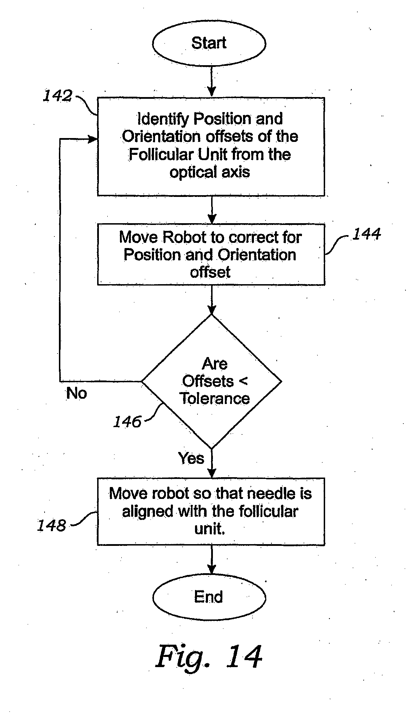

Fig. 14 is a flow diagram of an iterative procedure for aligning (both position and orientation) an elongate instrument used for harvesting and/or implanting hair follicles with a selected hair follicular unit.

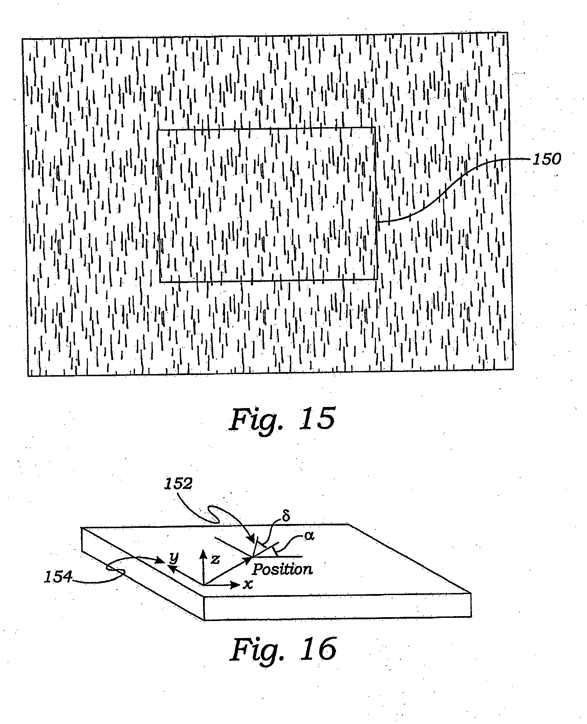

Fig. 15 depicts a camera image of hair follicular units in a region of interest on a human scalp.
Fig. 16 illustrates the position and orientation, i.e. defined by x,y offsets and in-plane and out-of-plane angles, of a hair follicular unit relative to the camera reference frame.

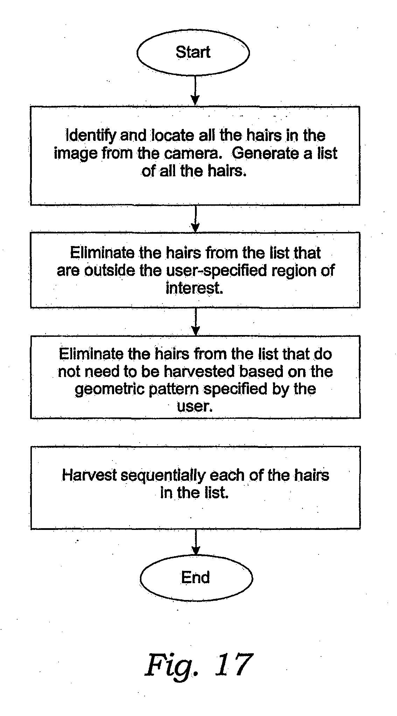

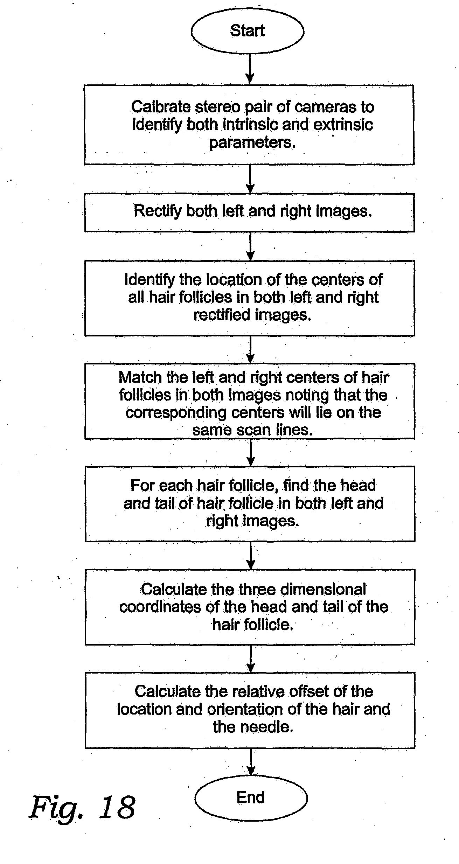

Fig. 17 is a flow diagram of an automated procedure for identifying a position and orientation of each of a multiplicity of follicular units in a region of interest on a human scalp, and then harvesting some or all of the identified follicular units. Fig. 18 is a flow diagram of an algorithm that uses images acquired from a stereo pair of cameras for identifying follicular units in a region of interest, and then computes the respective locations and orientations of the identified follicular units.

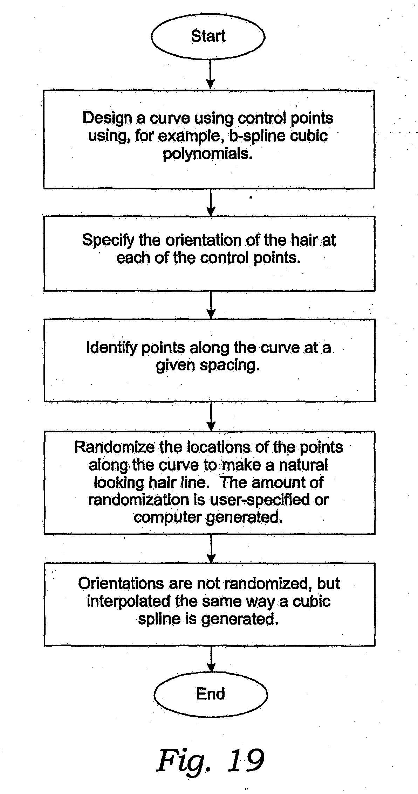

Fig. 19 is a flow diagram of an algorithm using control points to design a natural looking (implanted) hairline.

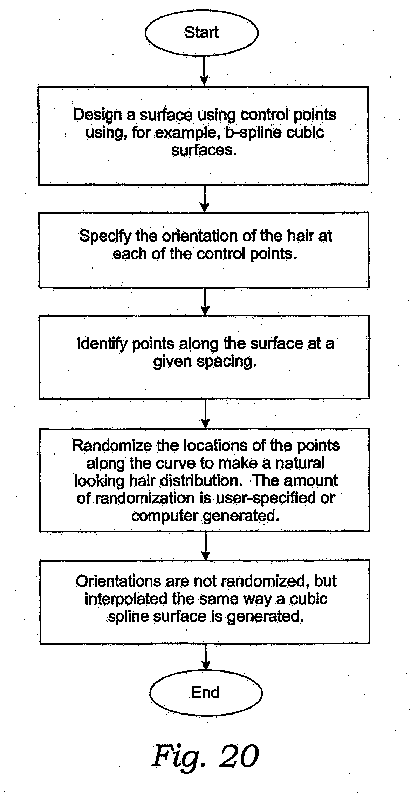

Fig. 20 is a flow diagram of an algorithm using control points to provide natural- looking randomness to implanted hair graft locations.

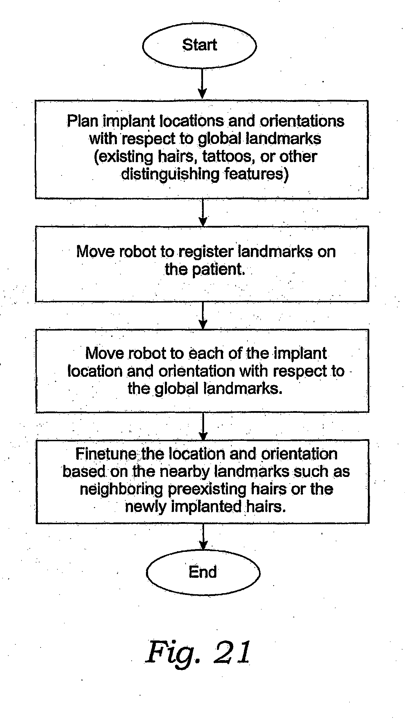

Fig. 21 is a flow diagram illustrating an automatic guidance feature of an image- guided robotics system.

### DETAILED DESCRIPTION OF THE ILLUSTRATED EMBODIMENTS

Fig. 1 depicts an image-guided robotics system 25, including a robotic arm 27, with a tool assembly 30 attached to a distal tool plate 20. The robotic arm 27 is preferably programmable and of a type such as those manufactured and distributed by Adept Technology, Inc. (www.adept.com). Another source of robotic arm assemblies suitable for embodiments of the invention are manufactured and distributed by Kuka Robot Group (www.kuka.com). The robotic arm 27 provides precisely controlled movement of the distal end plate 20 in six degrees of freedom (x, y, z, [omega], p, r), as is well-known in the art. Such movement of the distal plate is provided with a high degree of repeatability and accuracy (e.g., to 20 microns) by respective motors and encoders located in respective arm joints 21 of the robotic arm 27.

A variety of different end-effecter tools and/or assemblies may be attached to the distal end plate on the robotic arm 27 for performing various procedures on a human or animal patient. By way of example, the tool assembly 30 shown in Figs. 1-2 is designed for the harvesting and implantation of hair follicles from/in a human scalp or other body surface, and includes coaxially disposed harvesting and implanting cannulas 38 and 36, respectively, extending from a tubular extension 24 of a housing 22. The cannulas 36 and 38 are axially stiff, e.g., made of a hard metal or plastic, and thin-walled to facilitate tissue penetration. The implanting cannula 36 preferably has a needle-like tissue piecing tip, and the harvesting cannula preferably has a tissue-coring (e.g., serrated) tip. The robotic arm 27 automatically and precisely positions the respective harvesting and implanting cannulas 38 and 36 at desired locations, and in desired orientations, along a body surface (e.g., a scalp) of a patient based on control signals derived at least in part from image data acquired by one or more cameras 28 attached to the tool assembly housing 22.

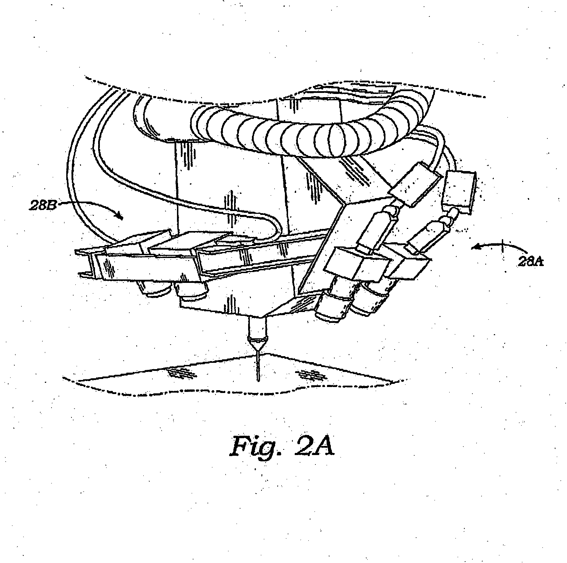

In particular, and as described in greater detail herein, movement of the robotic arm 27 is governed by a system controller (not shown), in response to control signals derived from image data acquired by a pair of "stereo" cameras 28 attached to the distal end of the robotic arm (proximate the tool assembly 30). In alternate embodiments, only a single camera need be used for image acquisition. Alternately, as depicted in Fig. 2A, and as described in greater detail herein, multiple pairs of stereo cameras 28A and 28B may be used in order to capture differing (i.e., broader and narrower) fields-of-view. In further embodiments, a single camera may be used to capture a first (i.e., broad) field-of-view, and a second camera may be used to capture a second (i.e., narrow) field-of-view. Other camera configurations are also possible.

Image data acquired by the camera(s) 28 is processed in a computer (not shown in Fig. 1) associated with the robotics system 25, which provides control signals to the system controller for directing movement of the robotic arm 27. In particular, images are acquired from each camera of the pair 28 at a desired magnification (e.g., in a range of 6x to 10x in one embodiment) and duty cycle (e.g., 30 hertz in one embodiment). The acquired images are digitized using known image segmentation techniques implemented in software on the computer in order to identify the position(s) and orientation(s) of objects of interest. In the case of procedures involving the removal or implantation of hair follicles, it may be desirable to die the hair follicles of interest with a dark color prior to a procedure, in order to increase the effectiveness of the image processing techniques. It may also be desirable to cut the hair follicles in the region(s) of interest to a substantially uniform length prior to the procedure.

As will be appreciated by those skilled in the art, one can visualize below the skin surface by adjusting the lighting, filters on the cameras, and various image processing techniques. This is because the reflection and absorption of light by the skin surface will change based on the wavelength of light used. Further, the depth of penetration of the light itself into the skin also varies based on the wavelength. Understanding these basic properties of light, images of the subcutaneous portions of the follicular units (hair follicles) may be obtained using appropriate respective wavelengths of light, including both visible light spectrum and infrared, capturing the different wavelengths of light using different imaging filters, and subtracting and/or combining images during image processing. This approach enables one to visualize the hair shaft of the follicular unit, both outside the skin, as well as under the skin surface, including all the way down to the bulb.

More particularly, the robotics system 25 is able to precisely track movement of the distal end plate (and end-effecter tool or assembly) in each of the six degrees of freedom (x, y, z, [omega], p, r) relative to three different reference frames. A "world frame" has its x,y,z coordinate origin at a center point of the base 29 of the robotic arm 27, with the x-y coordinates extending along a plane in a surface of a table 23 on which the base 29 of the robotic arm 27 is attached. The z-axis of the world frame extends orthogonally to the table surface through a first section of the robotic arm 27. A "tool frame" has its x,y,z coordinate origin established at the distal end tool plate. Lastly, a "base frame" may be registered relative to the world and tool frames. Each camera also has a (two-dimensional) camera coordinate system ("camera frame"), in which the optical axis of the camera ("camera axis") passes through the origin of the x,y coordinates. By aligning the respective world frame, tool frame, base frame and camera frames, the system controller can precisely position and orient an object secured to the tool plate (e.g., a needle) relative to another object, such as a hair follicular unit extending out of a patient's skin surface.

In order to physically align the camera axis with an axis of an end-effecter tool (e.g., an elongate needle cannula) fixed to the distal tool plate of the robotic arm 25, it is of practical importance to be able to calibrate, and thereby have the information to compensate for, the positional and rotational offsets between the end effecter "tool axis" and the camera axis, as well as the deviation from parallel of these respective axes. As an initial matter, the proximal base 29 of the robotic arm 27 is mounted to the table surface 23, so that the table surface 23 is aligned with the x-y coordinate plane of the world frame of the robotic system. Thus, a point lying anywhere on the table surface has a x-y coordinate location in the world frame, which can be identified in terms of x and y offset values (e.g., measured in mm) from the origin of the world frame located at a center point of the robotic arm proximal base interface with the table surface 23, with the z coordinate location of the point in the world frame equal to zero.

With reference to Fig. 13, an example of such calibration procedure is as follows: At step 160, the camera axis of a single camera fixed to the distal end tool plate of the robot arm 27 is aligned with a fixed "calibration point" located on the table surface 23. The base frame of the robotic system is then initiated, meaning that the origin of the base frame is set at the "calibration point" and the camera axis is aligned with the calibration point on the table surface. This initial position is called "home" position and orientation, and the robot arm 27 always starts from this position, even in the absence of the calibration point. At step 162, a scaling and orientation of the camera image relative to the base frame is then determined by first moving the robotic arm 27 (and, thus, the camera) a fixed distance (e.g., 5 mm) along the x axis of the base frame, so that the calibration point is still captured in the resulting image, but is no longer aligned with the camera axis. Because the camera frame x-y axes are not aligned with the base frame x-y axes, movement along the x axis of the base frame results in movement in both the x and y directions in the camera frame, and the new location of the calibration point is measured in the camera frame as a number of image pixels in each of the x and y directions between the pixel containing the relocated camera axis and the pixel containing the calibration point.

This process is repeated by moving the robotic arm 27 (and camera) a fixed distance (e.g., 5 mm) along the y axis of the base frame, and again measuring the x,y offsets in the camera frame of the new location of the calibration point. As will be appreciated by those skilled in the art, these measurements allow for scaling the physical movement of the robot/camera (in mm) to movement of an object in the camera image (in pixels), as well as the in-plane orientation of the x-y axes of the camera frame relative to the x-y axes of the base frame. It will further be appreciated that the scaling and orientation process of steps 160 and 162 are repeated for each camera in a multiple camera system, whereby variances in image movement between respective cameras may also be determined and calibrated.

At step 164, once the camera frame is calibrated with respect to the base frame, the camera axis is again aligned with a fixed calibration point lying on the surface of table 23, wherein the base frame is returned to is "home" position and orientation (0,0,0,0,0,0). The robotic arm 27 is then moved in one or more of the six degrees of freedom (x, y, z, [omega], p, r), so that an end effecter tool (e.g., needle tip) attached to the tool plate contacts the calibration point. By precisely tracking the movement of the robotic arm 27 from the initial home position/orientation of the tool frame to its position/orientation when the tool tip is contacting the calibration point, the system controller calculates the translational and rotational offsets between the initial home position and the camera axis. Because the camera is fixed to the tool plate, the measured offsets will be constant, and are used throughout the procedure for alignment of the tool frame with the camera frame (and, by extension, the base frame).

As will be described in greater detail herein, when using a stereo pair of cameras, e.g., camera pair 28 in Fig. 1, the respective optical axes (and camera frames) of the cameras are typically not installed or maintained in parallel, but are slightly verged, e.g., about ten degrees, which may be compensated for through known image processing techniques. In particular, the respective camera frames are aligned to have a common x (horizontal) axis, whereby a position and orientation (including in-plane depth) of objects captured in the parallel images may be aligned using image-processing techniques. One advantage of using a stereo camera pair 28 is that a "depth" in the camera frame of an identified object may be calculated based on the differences of the x,y position offsets of the object in the respective (left v. right) camera frames. In particular, the depth of implantation of a hair follicular unit ("graft") is important to the aesthetic result and is a challenge to achieve manually, particularly with the operator fatigue that can result when a large number of grafts are implanted. If the graft is implanted too deep, a divot-like appearance results; if implanted too shallow, a bump results or the graft may not stay in position.

In order to calculate a depth of a selected object, such as a hair follicular unit, the left and right images obtained from the stereo camera pair must first be aligned. Because the respective camera images are aligned horizontally, the same objects will appear in the same horizontal scan lines of the two images. And, because the depth of an object being imaged relative to the camera lenses is within a known range (e.g., established by the focal lengths of the respective cameras), a selected object in a first image (e.g., a hair follicular unit) can be matched to itself in the second image (to thereby align the images with each other) by calculating an effective depth of the object when paired with the possible candidate objects in the second image (i.e., in the same scan line) to determine which "pair" has a calculated depth in the possible range.

Another advantage of using a stereo camera pair 28 is the ability to obtain image data regarding the position and orientation of an end-effecter tool (e.g., a hair follicular unit harvesting cannula 38 shown in Figs. 1-2) in a same reference frame that image data is obtained regarding the position and orientation of objects of interest (e.g., hair follicles, wrinkle lines, tattoos, moles, etc.) on the skin surface. The respective left and right camera frames are calibrated with the tool frame in the same manner as described above for a single camera frame. Once these offsets are established, the relative positions and orientations of the end-effecter tool and objects on the skin surface (e.g., hair follicular units) may be determined and tracked in the tool frame.

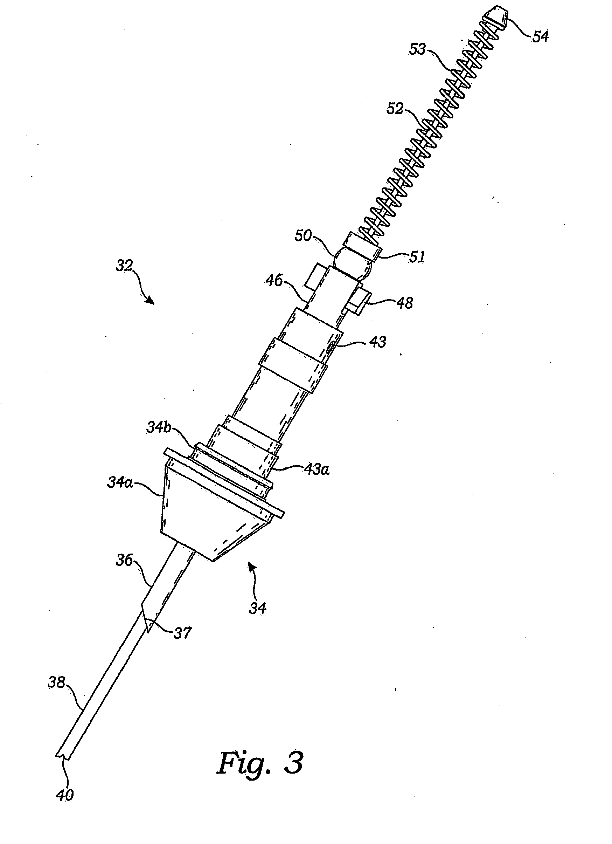

Fig. 14 is a simplified flow diagram of a procedure according to one embodiment of the invention for aligning the position and orientation of an elongate axis of the follicular unit harvesting cannula 38 with an elongate shaft axis of a hair follicular unit extending from the scalp, using only a single camera for image acquisition. As described in greater detail below, the harvesting cannula 38 generally comprises a hollow, tubular cannula having a serrated distal end for puncturing the epidermis and dermis immediately around an outer circumference of a follicular unit in order to envelop, capture and remove the entire follicular unit from the fatty subcutaneous tissues underlying the dermis, e.g., by rotating the cannula 38 in a drill-like motion, or by a quick reciprocating thrust along its longitudinal axis. The harvesting cannula 38 may be advanced and withdrawn by its own longitudinal motion (i.e., relative to the tool plate to which it is attached), or by longitudinal motion of the robotic arm 27, or by a combination of both, in order to core and remove the respective follicular units, e.g., by friction and/or with the aid of a weak vacuum. For example, the tool assembly 30 may have its own controller and actuation system separate from the robotics system 25. A more detailed description of the follicular harvesting and implantation tool assembly 30 is provided below, in conjunction with the description of the embodiments of Figs. 3-11. It should also be appreciated that the positioning and orientation process used for aligning the elongate axis of the harvesting cannula 38 with the elongate axis of a hair follicular unit will have much broader applicability than just for hair removal and/or implantation procedures. By way of non-limiting examples, substantially similar positioning and orientation procedures may be used for aligning a laser, or an injection needle, with desired physical features and/or locations on a patient's skin surface in a timely and precise manner.

After the robotics system 25 has been initiated and calibrated so that the camera frame is aligned with the tool frame (described above in conjunction with Fig. 13), image data is acquired and processed by the system computer to identify objects of interest in the camera frame. By way of example, Fig. 15 depicts a camera image of hair follicular units in a region of interest 150 on a human scalp. From images of this region of interest 150, image segmentation and screening software residing in the computer identifies and selects one or more particular follicular units of interest for harvesting from the scalp. With reference to Fig. 16, a position of a selected hair follicular unit 152 is identified in terms of its x,y offset coordinates in the camera frame (the z axis being the camera optical axis which is preferably aligned substantially orthogonal to the surface of the scalp at the region 150). Unless the camera axis happens to be exactly aligned with the longitudinal axis of the follicular unit 152 (in which case the follicular unit will appear as a circular point representing an end view of the hair shaft), the image of follicular unit will be in the form of an elongate line having an "apparent" length that will depend on the angle of the camera frame relative to the follicular unit. Because of physical attributes of a hair follicular unit, its base (i.e., the end emerging from the dermis) can be readily distinguished from its tip as part of the image segmentation process. For example, the base portion has a different profile and is generally thicker than the distal tip portion. Also, a shadow of the follicular unit can typically be identified which, by definition, is "attached" at the base.

The x,y locations of the follicular unit base in the camera frame are then calculated and represent the position offsets of the hair base. Orientation offsets of the follicular unit 152 are also calculated in terms of (i) an in-plane angle [alpha] formed by the identified follicular unit shaft relative to, and in the same plane as, the x (or y) axis of the camera frame; and (ii) an out-of-plane angle [delta] that is an "apparent" angle formed between the follicular unit shaft and the scalp, i.e., between the follicular unit and the plane of the x,y axes of the camera frame. As noted above, the hair shaft is preferably trimmed prior to the procedure to a substantially known length, e.g., 2 mm, so the out-of-plane angle [delta] may be calculated based on a ratio of a measured apparent length of the image of the follicular unit to its presumed actual length, which ratio is equal to the cosine of the out-of-plane angle [delta].

Returning to Fig. 14, at step 142, the x,y position and orientation offsets are identified for a selected hair follicular unit, as described above. The computer then calculates the necessary movements of the robotic arm 27 to cause the camera axis to be aligned in the same position and orientation of the calculated offsets. The base frame and tool frame are also "moved" by the same x,y and rotational offsets (i.e., until angles [alpha] and [delta] are both equal to 0), so that the camera, base and tool frames remain aligned at the new position and orientation of the camera axis. Because of the inherent possible variances and errors in the system and in the assumptions (e.g., regarding the hair follicular unit length) the actual position and orientation of the hair follicular unit may not match the calculated values. Thus, once the robotic arm 27 (and camera axis) is moved by the calculated positional and rotational offsets, the follicular unit is again imaged and (at step 146) a determination is made as to whether the camera axis is aligned with the position and orientation of the follicular unit within acceptable tolerances. If the camera axis is adequately aligned with the follicular unit, the robotic arm 27 is moved a last time (at step 148) in order to align the harvesting cannula 38 in the "confirmed" position of the camera axis (i.e., based on the offsets obtained in the above-described calibration process). However, if the (in step 146) the camera axis is not adequately aligned with the hair follicular unit, the procedures in steps 142-146 are repeated, starting from the new camera axis location.

As will be appreciated by those skilled in the art, in embodiments of the invention, the duty cycle of the image acquisition and processing is substantially faster than the movement of the robotic arm 27, and the process of identifying and calculating position and orientation offsets of selected hair follicular units relative to the camera axis can effectively be done "on- the-fly," as the robotic arm is moving. Thus, the end destination (i.e., position and orientation) of the robotic arm 27 (and harvesting cannula 38) may (optionally) be constantly adjusted (i.e., fine tuned) as the harvesting cannula 38 is moved into alignment with the follicular unit. Because such adjustments begin immediately, movement of the robotic arm 27 is more fluid and less jerky. This iterative feedback process, referred to as "visual- servoing," continually calculates and refines the desired position and orientation of the harvesting cannula 38, in order to minimize the image of the hair follicular unit, i.e., until the image transforms from a line to a point.

Thus, the image-guided robotics system 25 may be used to perform automated or semi-automated procedures for identifying position and orientation of a large number of hair follicular units in a region of interest on a patients scalp, and then accurately harvest some or all of the follicular units. One or more cameras attached to the working distal end of the robotic arm capture images at a desired magnification of a selected area of the patient's scalp. A computer system processes the images and identifies (through known thresholding and segmentation techniques) the individual hair follicular units, as well as their respective positions and orientations relative to the camera frame. Through a user-interface (e.g., a display and a standard computer mouse), an attending surgeon may define a region on the scalp from which hair follicular units are to be harvested and defines a harvesting pattern, such as, e.g., taking every other hair follicular unit in the region, leaving a defined number of follicular units between harvested follicular units, taking a certain percentage of follicular units, leaving behind an aesthetically acceptable pattern, etc.

For example, images obtained from a wide field-of-view pair of stereo cameras may be used by the attending physician to locate generally a region of interest, while images obtained from a narrow field-of-view pair of stereo cameras are used to accurately guide the harvesting tool with the individual selected follicular units. Once the hair follicular units to be harvested have been identified, the robotics system systematically aligns a harvesting tool (e.g., harvesting cannula 38) with each hair to be harvested; the respective hair follicles are harvested, and the process is repeated for all of the selected follicular units in the defined harvest region. It will be appreciated that in some cases, the individual hair follicular units being harvested are then implanted in another portion of the patient's scalp, whereas in other instances the harvested hair follicular units are discarded. It will also be appreciated that, rather than a coring harvesting tool, such as cannula 38, another type of hair removal end- effecter tool may be employed, such as, e.g., a laser. It will be still further appreciated that the above-described techniques for aligning the camera frame with the robot tool frame for precisely aligning an end-effecter tool may be equally applicable to other types of end- effecter tools, such as an injection needle (or a plurality of injection needles) used for injecting ink for forming tattoos on a skin surface of a patient.

Fig. 17 is a flow diagram of an automated (or semi-automated) procedure for identifying a position and orientation of all follicular units in a region of interest on a patient's scalp, and then accurately harvesting some or all of the identified follicular units. Fig. 18 is a flow diagram of a procedure using a stereo pair of cameras to identify individual follicular units in a region of interest on a patient's scalp, and then compute a location and orientation of each in the respective camera frames and robot tool frame. The procedure starts by calibrating the stereo pair of cameras to identify both intrinsic and extrinsic parameters, in accordance with well known techniques. Intrinsic parameters are intrinsic to the individual camera, such as internal optics, distortion, scaling, and the like. Extrinsic parameters relate to characteristics between the two cameras, e.g., differences in the alignment of their respective optical axes (which are ideally parallel to one another, but as since this is unlikely as a practical matter, mathematical compensation is required). Calibration of intrinsic and extrinsic parameters is well known in the field of stereo imaging and will not be explained in detail herein.

As discussed above, the locations of the centers of the hair follicles are identified and matched in both the left and right rectified images. The head and tail of each hair follicle is then identified in both the left and right images, wherein the three dimensional coordinates of the head and tail of the hair follicle may be calculated. Finally, the relative offset of the location and orientation of the hair follicle and the cannula are determined by employing the images of the cameras which see both the cannula and the hair follicle, in accordance with well known stereo imaging techniques.

The aesthetic result of a hair transplant procedure depends in part on implanting the grafts in natural-looking patterns. The computer can efficiently "amplify" the surgeon's skill by "filling in the blanks" among a small fraction of the implant sites for which the surgeon determines graft location and orientation. Achieving a natural-looking hairline is particularly important for a good aesthetic result. Instead of painstakingly making incisions for all of the near-hairline implant sites, the surgeon indicates a few hairline implant locations and orientations and the computer fills in the rest by interpolating among the designated sites, using the imaging system to identify and avoid existing follicular units. Fig. 19 illustrates an algorithm using control points to design natural looking hairline. A curve is designed using control points based on, for example, b-spline cubic polynomials. The control points are specified by the operator. The orientation of the hair at each of the control points is specified. Points along the curve are identified at a given spacing, for instance, by interpolation. The locations of the points along the curve may be randomized to make a natural looking hair line. The amount of randomization may be user-specified or computer-generated. It is preferable that the follicular unit orientations are not randomized but are interpolated, for example, the same way a cubic spline is generated. Randomization of the location and interpolation of the orientation create more natural looking implants.

Natural looking randomness is important in both the critical hairline region and in the balance of the recipient sites. This can be achieved using the procedure illustrated in Fig. 20, wherein a surface is designed using control points based on, for example, b-spline cubic surfaces. Again, the orientation of the hair at each of the control points is specified. Implant points along the surface are identified at a given spacing. The locations of the points along the surface may be randomized to make a natural looking hair distribution. The amount of randomization may be user-specified or computer-generated. Again, the orientation of the respective follicular units is preferably not randomized, but interpolated the same way a cubic spline surface is generated. Randomization and interpolation schemes are known in the art, and can be adapted for this method.

It is often desirable to leave the existing hair in the recipient region at its natural length, which can interfere with the vision system's access to individual recipient sites. This can be overcome by a gentle air jet directed at the recipient site, causing the hair in that region to be directed away from the target site. If necessary, the hair can be dampened to facilitate this step. The air jet also can disperse blood that emerges from the incised recipient site, thus maintaining visual access during graft implantation. Such an air jet can be part of a more complex tool assembly attached to the robotic arm tool plate.

The robotics system 25 uses real-time information from the vision system to monitor the position of the patient (typically using fiducial markers in the recipient region of the scalp), of the implanting tool, and of existing follicular units to guide the implanting tool into place for incising the recipient site and implanting the graft. Fig. 21 shows an example of the automatic guidance feature of the robotic system, including the step of planning implant locations and orientations with respect to global landmarks (e.g., existing hairs, tattoos, or other distinguishing features). The robot is then moved to register landmarks on the patient. The register information can be stored in memory for reference. The robot can make use of the registered landmarks as reference points for recognizing its position relative to the working surface. The robot is moved to each of the implant location and orientation with respect to the global landmarks. The global landmarks provide a global reference for global movements. The location and orientation are fine-tuned based on the nearby landmarks such as neighboring preexisting hairs or newly implanted hairs. The nearby landmarks provide a local reference for local movements.

Hair transplantation generally includes three steps: follicular unit harvesting, recipient site incision, and follicular unit placement in the incision. Fig. 3 shows one embodiment of a three-part tool 32 used for performing all three of these functions. Although the ensuing description of the three-part tool 32 is with reference to its use as part of the tool assembly 30 carried on the robotic arm 27 in the system 25 of Fig. 1, it will be appreciated that hand-held and operated embodiments of the three-part tool 32 are also possible. More particularly, the three-part tool 32 includes an outer ("implanting") cannula 36 having an open, tissue-piercing (e.g., beveled) distal end 37 used for making incisions at recipient (implantation) sites in a body surface. An inner ("harvesting") cannula 38 is coaxially positioned in an interior lumen of the implanting cannula 36, and has an open, tissue-coring (e.g., rough or serrated) distal end 40. The harvesting cannula 38 has an interior lumen appropriately sized for harvesting singular human hair follicular units by coring the respective follicular units and extracting them from a body surface (typically but not necessarily a scalp).

By way of non-limiting examples, embodiments of the harvesting cannula 38 may have interior lumens that range from approximately 0.3 millimeters to 2.0 millimeters in diameter. In one embodiment, the harvesting cannula lumen has a diameter that is approximately 1 millimeter in diameter. Notably, different sized harvesting cannulas 38 may be used for harvesting single-follicle follicular units than for harvesting multi-follicle follicular units. In either case, an inner wall surface of the harvesting cannula lumen may be textured to facilitate frictional grasping the respective follicular units for extraction from the body surface after they are cored.

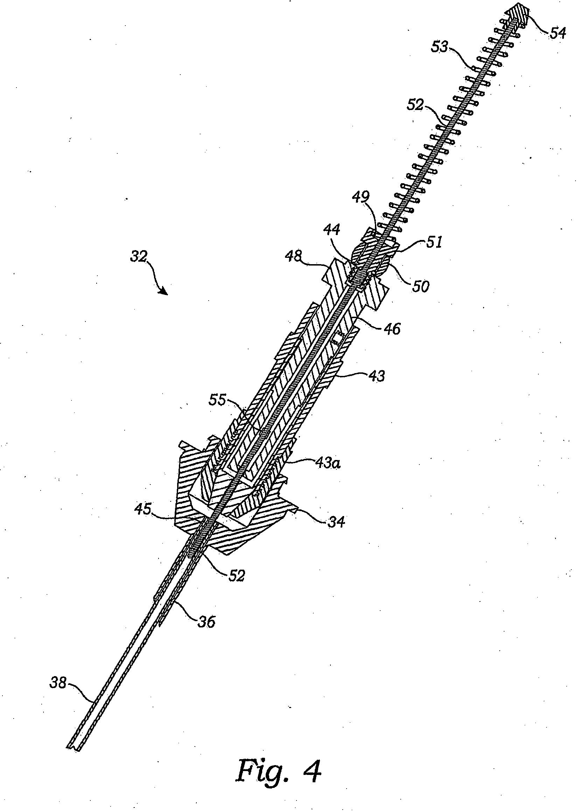

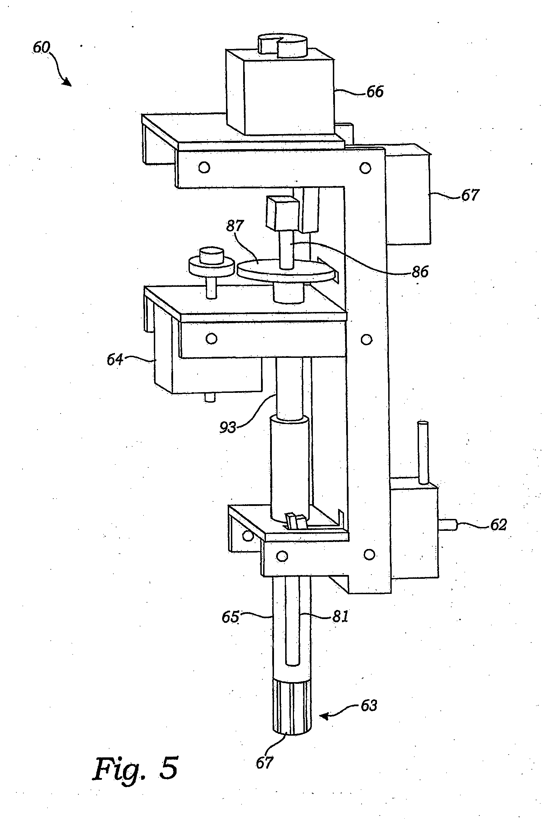

With reference also to Figs. 4 and 5, the tool assembly 30 includes a motor drive assembly 60 mounted in the housing 22 and configured to receive and operatively engage the component parts of the three-part tool 32. In particular, the implanting cannula 36 is fixedly attached to a proximal hub 34, including a distal facing tapered portion 34a and a proximally directed engagement portion 34b. The engagement portion 34b may be detachably-coupled (snap-fit) with a resilient gripper 63 extending from a tubular sleeve 65 in the motor drive assembly 60. In the illustrated embodiment, the gripper 63 comprises a plurality of resilient arm members 67 that are attached to or integral with, the tubular sleeve 65. It will be appreciated that other detachable coupling mechanisms may be employed in alternate embodiments. The tubular sleeve 65 engages a rack-and-pinion drive mechanism 81 driven by a first motor 62 of the motor drive assembly 60, so that, when the hub 34 is coupled to the gripper 63, the motor 62/drive mechanism 81 provide axial (i.e., reciprocating) motion of the implanting cannula 36 relative to the harvesting cannula 38 (and also relative to the tool assembly housing 22/24).

The harvesting cannula 38 extends proximally through a bore 45 of the implanting cannula hub 34 and implanting cannula 36, and is fixedly attached to distal chuck portion 43a of a pin vise 43 seated in, and rotatable relative to, a bore of hub 34. An elongate body 46 is seated in, and fixedly attached to, the pin vise 43, and includes one or more radially-outward extending flanges 48 that engage a corresponding set of slots (not shown) in a distally projecting tubular drive member (not shown - extends internally through housing 93) coupled to an output gear 87 driven by a second motor 64 of the motor drive assembly 60 for thereby rotating the respective elongate body 65 and harvesting cannula 38, respectively, about a longitudinal axis of the harvesting cannula 38. As will be appreciated, a belt drive or other means for rotating the tubular drive member (and, thereby, the harvesting cannula 38) may be used in alternative embodiments. The elongate body 46 further includes a recessed section 44 located proximally of the flanges 48, which seats an annular retaining member 50 for detachably-coupling (via a snap-fit type connection) with the tubular drive member (proximal of the slots that engage flanges 48), thereby retaining the harvesting cannula 38 in position when the tool 32 is coupled with the motor dive assembly 60.

An elongate obturator 52 is slidably positioned in an interior lumen of the harvesting cannula 38, and has a proximal end attached to a seating member 54 that engages with a linear ("screw-drive") drive mechanism (not shown) driven by a third motor 66 of the motor drive assembly 60 for selectively providing a distally-directed, "pushing" force on the obturator 52 relative to the harvesting cannula 38. A spring 53 is seated in an annular recess 49 formed in a proximal end-cap 51 of the elongate body 46, and extends (over the obturator 52) to the distal side of the seating member 54. The spring 53 applies a proximally-directed, "pulling" force on the seating member, to thereby bias the obturator against the screw drive.The drive motor assembly further includes a "release" motor 67, that applies a distally-directed (pushing) force against the end-cap 51 via a tubular release member 86, which causes the respective attachment couplings (i.e., the implanting cannula hub 34b and gripper 63, and the harvesting cannula retaining member 50 and the tubular drive member) to decouple for removal of the tool 32 from the tool assembly 30, e.g., for replacing one or both of the implanting and harvesting cannulas 36 and 38. In this manner, the multi-part tool 32 may be loaded into the tool assembly 30 by insertion (in the proximal direction) of the tool 32 ("back loaded") through the tubular extension 24 of the housing 22, until the respective couplings 34b and 50 snap into place with their counterparts in the motor drive assembly 60, and released by application of a sufficient force by the motor 67 on the release member 86 to decouple the respective couplings. A stop member 55 is attached to the obturator 52 that abuts the distal side of the end-cap as the release member 86 applies a downward force on the end-cap 51, so that the obturator 52 accompanies the rest of the tool 32 as it is released from the motor drive assembly 60 (and from the tool assembly 30).

The motor drive assembly 60 further comprises control circuitry for controlling operation of the respective motors 62, 64, 66, and 67. The control circuitry may include an independent processor (not shown) associated with the motor drive assembly 60, which receives as inputs information from the robotic system 25, including but not limited to positioning data obtained from images acquired of the respective cannulas 36, 38 and body surface/objects (e.g., hair follicles). Additionally or alternatively, a respective encoder may be operatively coupled with one or more of the motors 62, 64, 66, and 67 for tracking the relative movement and, thus, position information, of the implanting cannula 36, harvesting cannula 38, and/or obturator 52.

For harvesting a follicular unit from a body surface (e.g., a scalp), the robotic arm 27 positions and aligns the harvesting cannula 38 with a longitudinal axis of a selected follicular unit to be harvested. The harvesting cannula 38 is then advanced over the selected follicular i unit by motion of the robotic arm 27, accompanied by simultaneous rotational movement of the harvesting cannula 38 about its longitudinal axis by the motor 64, with the open distal end 40 of the cannula 38 penetrating the body surface into the subcutaneous fatty layer surrounding and underlying the follicular unit. In alternate embodiments, a linear drive mechanism may be additionally provided in the motor drive assembly 60 for providing independently controlled axial translation of the harvesting cannula 38 relative to the tool assembly housing 20 (and implanting cannula 36). The harvesting cannula 38 is then withdrawn from the body surface by motion of the robotic arm 27 to thereby extract the follicular unit, which is carried in the lumen of the harvesting cannula. In some embodiments, a vacuum source may be selectively placed in communication with the harvesting cannula lumen to apply a proximally-directed "pulling" force to facilitate grasping and extracting the follicular unit, as well as to help retain the follicular unit in the harvesting cannula lumen after it has been harvested.

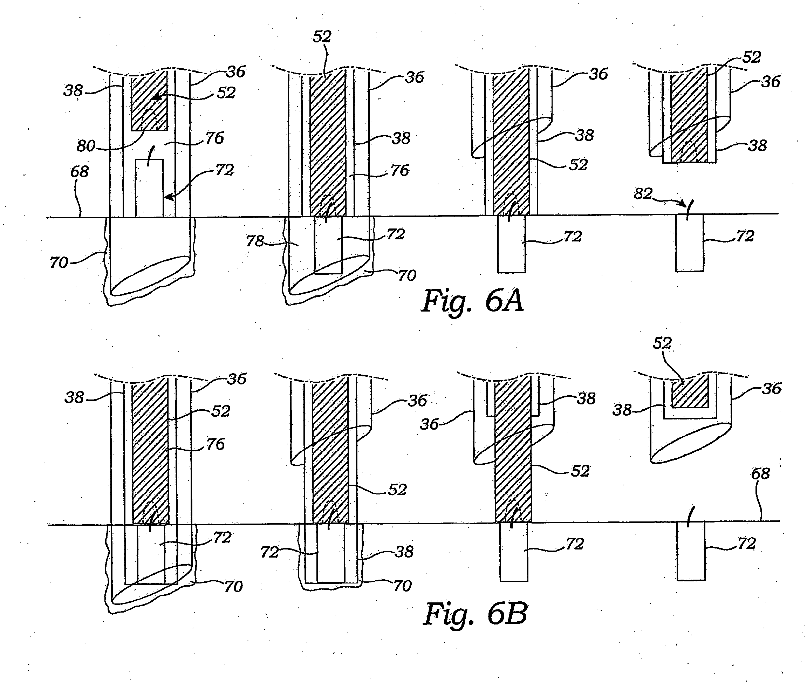

For implantation, the tool assembly 30 is repositioned by the robotic arm 27 to a selected implantation site on the body surface. At the implantation site, a longitudinal axis of the implanting cannula 36 is preferably aligned with a desired orientation of the follicular unit, when implanted. With reference to Figs. 6A-B, the tissue-piercing distal end 37 of the implanting cannula 36 is advanced over the harvesting cannula 38 and into the body surface 68, creating a subcutaneous implantation cavity 70 of an appropriate depth and size for receiving the harvested follicular unit 72. This puncture motion by the cannula 36 is automatically controlled by motor 62, and is preferably very rapid in order to minimize trauma to the tissue surface 74 of the implantation cavity 70.

In one embodiment (shown in Fig. 6A), the follicular unit 72 is moved axially by the obturator 52 (under the control of motor 66) from the harvesting cannula lumen 76, where it has remained undisturbed since it was harvested, into a distal end portion of the implanting cannula lumen 78. This repositioning of the follicular unit 72 from the harvesting cannula lumen 16 into the implanting cannula lumen 78 may take place before, during, or after the implanting cannula 36 has punctured the body surface 68. The obturator thereafter maintains the follicular unit 72 in the implantation cavity 70 as the implanting cannula 36 is withdrawn from the body surface 68 by translational movement relative to the obturator 52. Once the implanting cannula 36 is withdrawn, the obturator 52 is also withdrawn, with the follicular unit 72 implanted in the body surface. A distal facing end 80 of the obturator 52 is preferably recessed to allow room for one or more hair follicles 82 protruding from the follicular unit 72.

In another embodiment (shown in Fig. 6B), the respective distal ends of the implanting and harvesting cannulas 36 and 38 are aligned (i.e., by relative movement of the implanting cannula 36) so that their respective distal ends 37 and 40 are approximately coextensive. This alignment of the respective cannula distal ends 37 and 40 may take place before, during, or after the implanting cannula penetrates the body surface to form the implantation cavity 70. Thereafter, the respective cannulas 36 and 38 are withdrawn from the implantation cavity 70, while the follicular unit 72 is retained therein, i.e., by simultaneous movement of the robotic arm 27 away from the body surface 68 and of the obturator 52 towards the body surface 68. In alternate embodiments having a linear drive mechanism in the motor drive assembly 60 for providing independently controlled axial translation of the harvesting cannula 38 relative to the tool assembly housing 20 (and implanting cannula 36), the respective cannulas 36 and 38 may be withdrawn from the implantation cavity 70 relative to (and without requiring simultaneous movement of) the obturator 52 by operation of the motor drive assembly 60. In other alternate embodiments, a source of pressurized air selectively placed in communication with the harvesting cannula lumen 76 may be used to retain the follicular unit 72 in the implantation cavity 70 as the cannulas 36 and 38 are withdrawn.

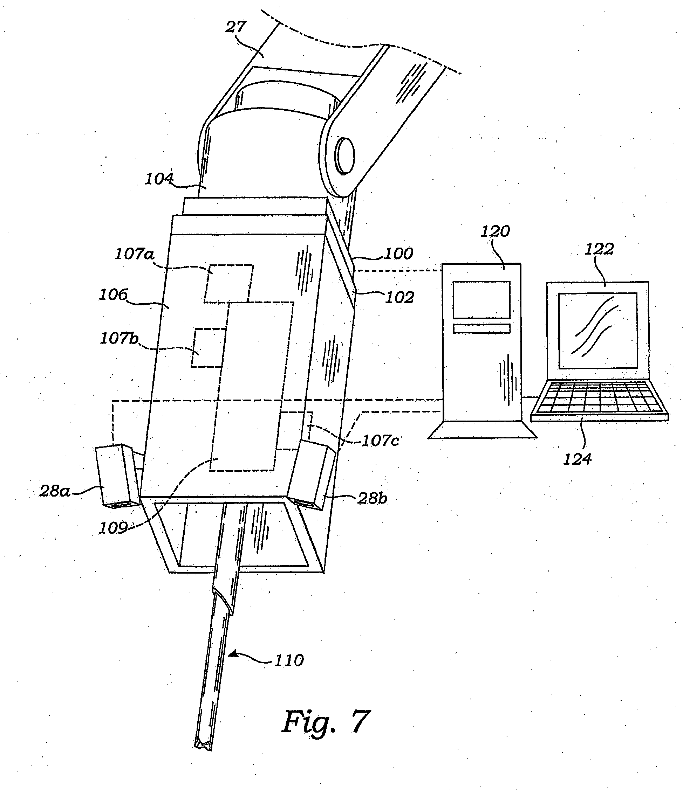

Fig. 7 illustrates a distal portion of the robotics system 25 in accordance with some embodiments of the invention. A force sensor 100 is secured to an arm 104, a plate 102 mounted to the force sensor 100, and a motor drive, or "positioning" assembly 106 secured to the plate 102. Alternatively, the plate 102 could be secured directly to the arm 104, in which cases, the force sensor 100 may be secured between the positioning assembly 106 and the plate 102. Alternatively, the force sensor 100 may be located within the positioning assembly 106. The force sensor 100 is configured to sense three forces Fx, Fy, Fz in three different orthogonal directions X, Y, Z, and three orthogonal moments Mx, My, Mz. In other embodiments, the force sensor 100 may be configured to sense one or two of the forces Fx, Fy, Fz, and/or one or two of the moments Mx, My, Mz. As shown in the figure, the force sensor 100 is coupled to a computer 120, which receives data from the force sensor 100 representing the sensed force(s) and/or moment(s). In other embodiments, the force sensor data may go directly to the robot.

During the above harvesting and implanting process, the force sensor 100 monitors one or more force/moment component transmitted from the positioning assembly 106. For example, the force sensor 100 may monitor a force Fz, which has a directional vector that is approximately parallel to an axis of a harvesting cannula 200. The sensed force Fz is transmitted to the computer 120, which determines whether a magnitude of the sensed force Fz is within an acceptable limit. In some embodiments, the computer 120 is configured (e.g., programmed) to stop a harvest process or an implant process if the sensed force Fz exceeds a prescribed limit, which may indicate that the harvesting cannula 200 or the implanting cannula 202 is pressing against the skull, for example. As such, the force sensor 100 provides a safety feature that prevents the harvesting cannula 200 and the implanting cannula 202 from injuring a patient in an unintended way.

Instead of, or in addition to, using the force sensor 100 as a safety feature, the force sensor 100 may also be used to control a positioning of the harvesting cannula 200 and/or the implantation cannula 202. As the harvesting cannula 200 is being advanced through the skin and into tissue underneath the skin, it experiences a force Fz, which represents a resistance encountered by the coring needle 200. Fig. 12 illustrates a force diagram that represents a force resistance Fz sensed by the harvesting cannula 200 as it is advanced through the skin and into tissue. Such force Fz is transmitted by the various components within the positioning assembly 106 to the force sensor 100, which measures such force Fz and transmits the force data to the computer 120. Because the skin surface is relatively tough, initially, as the harvesting cannula 200 pushes against skin, it will not immediately penetrates the skin, and will experience a force resistance Fz provided by the skin surface. The force resistance Fz increases from zero to a value Fp, at which point, the harvesting cannula 200 penetrates through the skin. Because the tissue underneath the skin is relatively softer than the skin, the force resistance Fz experienced by the harvesting cannula 200 will be less than Fp after penetration of the skin.

As shown in Fig. 12, after the value Fp is reached, the force curve falls back to a second value Fs, which represents the force resistance sensed by the coring needle 200 after it has penetrated the skin surface. The force Fz will continue to increase from that point as the harvesting cannula 200 continues to be advanced into the tissue. This is because as more portion of the harvesting cannula 200 is advanced into the tissue, it will contact more tissue that is underneath the skin, thereby increasing an amount of surface friction between the harvesting cannula 200 and the tissue. In some cases, if the harvesting cannula 200 hits a bone, the force diagram will result in a spike (shown in dotted line in the figure). The computer 120 may be programmed to monitor the force curve being generated as the harvesting cannula 200 is being advanced during the harvest process, and controls the harvesting cannula 200 based on the force curve. For example, in some embodiments, the computer 120 activates a positioner in the positioning assembly 106 to advance the harvesting cannula 200 at a first rate until a dip in the force curve is observed, indicating that the harvesting cannula 200 has penetrated the skin. The computer 120 then activates the positioner to advance the harvesting cannula 200 at a second rate until a desired penetration depth is accomplished. In some embodiments, the first rate may be faster than the second rate.

In the illustrated embodiments, the positioning assembly 106 includes a holding unit 109 for engagement with a cannula assembly 110, and a plurality of positioners 107a- 107c. The holding unit 109 is configured to engage with different parts of the cannula assembly 110 so that the cannula assembly 110, as a whole, can be positioned by the positioning assembly 106. The holding unit 109 also allows different components of the cannula assembly 110 to be controlled after the cannula assembly 110 is engaged with the holding unit 109. The positioners 107a- 107c are configured for moving different components of the cannula assembly 110 after it has been engaged with the holding unit. Although three positioners 107a- 107c are shown, in other embodiments, the positioning assembly 106 may include more or less than three positioners 107. In some embodiments, the positioning assembly 106 may include the motor drive assembly of Fig. 5, which includes three motors (positioners) for moving different components of the cannula assembly 110, plus an additional motor for disengaging the cannula assembly 110 from the positioning assembly.

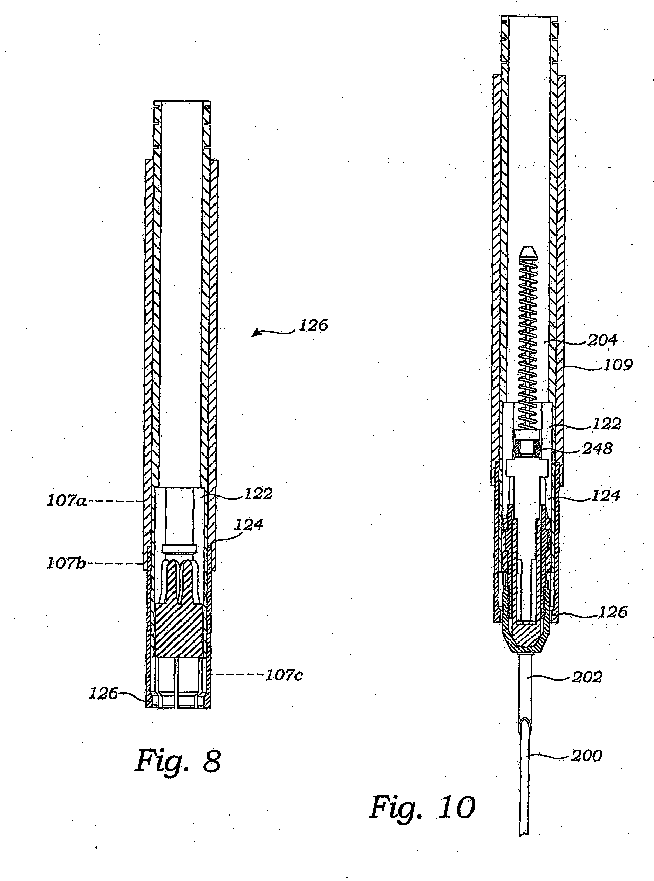

Fig. 8 illustrates a holding unit 109 constructed in accordance with some embodiments. The holding unit 109 includes a first engagement portion 122 for engaging a first portion of the cannula assembly 110, a second engagement portion 124 for engaging a second portion of the cannula assembly 110, and a third engagement portion 126 for engaging a third portion of the cannula assembly 110.

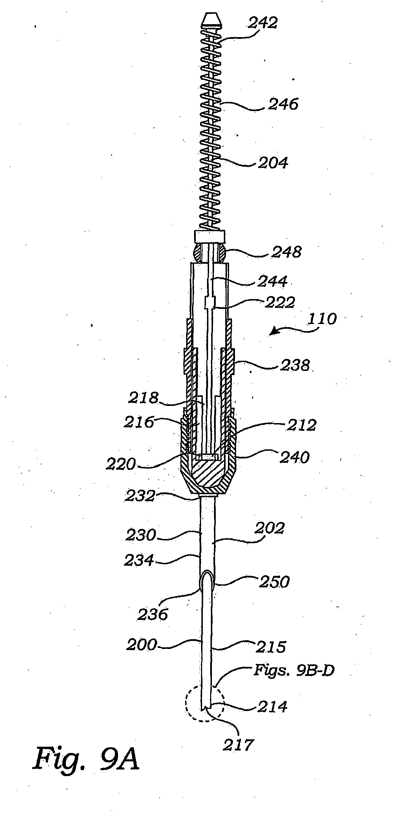

Fig. 9A illustrates the cannula assembly 110 in accordance with some embodiments. The cannula assembly 110 has a similar configuration as the tool 32 shown in Figs. 3-4. The cannula assembly 110 includes a harvesting cannula 200, an implanting cannula 202, and a plunger (obturator) 204. The harvesting cannula 200 has a proximal end 212, a distal end 214, a body 215 extending between the proximal and distal ends 212, 214, and a lumen 217 defined at least partially by the body 215. In the illustrated embodiments, the lumen 217 has a cross sectional dimension that is between 0.3 millimeter and 2.0 millimeters, and more preferably, approximately 1 millimeter. The cannula assembly 110 further includes a shaft 216 having a proximal end 218, a distal end 220, and a lumen 222 extending between the proximal and distal ends 218, 220. The proximal end 212 of the harvesting cannula 200 is secured to the distal end 220 of the shaft 216. The implanting cannula 202 has a proximal end 232, a distal end 234, a body 230 extending between the proximal and distal ends 232, 234, and a lumen 236 within the body 230. The lumen 236 has a cross sectional dimension sized for accommodating at least a portion of the harvesting cannula 200, and for allowing the harvesting cannula 200 to slide relative to the implanting cannula 202. The distal end 234 of the implanting cannula 202 has a sharp tip 250 for piercing tissue.

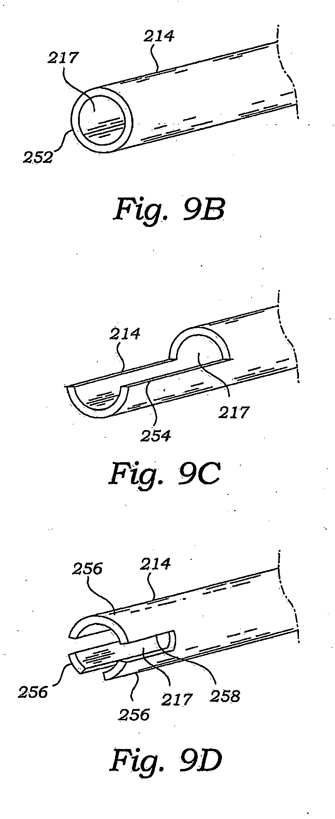

In the illustrated embodiments, the distal end 214 of the harvesting cannula 200 has a tubular configuration (Fig. 9B). In such cases, the edge 252 of the harvesting cannula 200 may have a sharp configuration for allowing the harvesting cannula 200 to penetrate tissue. In other embodiments, the distal end 214 of the harvesting cannula 200 may have an arc configuration (Fig. 9C). In such cases, the ends 254 of the arc portion may have a sharp configuration for allowing the harvesting cannula 200 to cut tissue as the harvesting cannula 200 is rotated about its axis. In further embodiments, the distal end 214 of the harvesting cannula 200 can include a plurality of cutting portions 256, with each cutting portion 256 having a sharp edge 258 for cutting tissue (Fig. 9D). It should be noted that the distal end 214 of the harvesting cannula 200 is not limited to the examples described previously, and that the distal end 214 can have other configurations in other embodiments, as long as it can core tissue.

The cannula assembly 110 further includes a first engagement portion 238 and a second engagement portion 240. The first engagement portion 238 has a tubular configuration, and is secured to the shaft 216. The second engagement portion also has a tubular configuration, and is secured to the proximal end 232 of the implanting cannula 202. proximal end 232 of the implanting cannula 202. The first and the second engagement portions 238, 240 are sized and shaped to engage with corresponding components of the holding unit 109. It should be noted that the first and second engagement portions 238, 240 are not limited to the example of the configuration illustrated, and that the engagement portions 238, 240 can have other configurations in other embodiments. For example, in alternative embodiments, the engagement portion 238 does not have a tubular configuration. In such cases, the engagement portion 238 can be a structure that is secured to, or extends from, a surface of the shaft 216. Similarly, in other embodiments, the engagement portion 240 can be a structure that is secured to, or extends from, a surface of the implanting cannula 202, and needs not have a tubular configuration. As shown in the figure, the cannula assembly 110 also includes a connector 248 secured to the shaft 216. The connector 248 has a shape that resembles a sphere, but may have other shapes in other embodiments.

The plunger 204 has a proximal end 242 and a distal end 244. The plunger 204 is at least partially located within the lumen 217 of the harvesting cannula 200, and is slidable relative to the harvesting cannula 200. The cannula assembly 110 further includes a spring 246 coupled to the plunger 204 for biasing the plunger 204 in a proximal direction relative to the harvesting cannula 200. In the illustrated embodiments, the plunger 204 is described as a component of the cannula assembly 110. In other embodiments, the plunger 204 is not a part of the cannula assembly 110. For example, the plunger 204 may be a component of the positioning assembly 106.

Fig. 10 illustrates the cannula assembly 110 that has been engaged with the positioning assembly 106. When the cannula assembly 110 is snapped onto the positioning assembly 106, the first engagement portion 122 of the holding unit 109 is engaged with the connector 248, the second engagement portion 124 is engaged with the first engagement portion 238 of the cannula assembly 110, and the third engagement portion 126 is engaged with the second engagement portion 240 of the cannula assembly. The connector 248 allows the cannula assembly 110 to be detachably secured to the positioning assembly 106. The first engagement portion 122 of the holding unit 109 is coupled to the first positioner 107a. In some embodiments, the harvesting cannula 200 is not translatable. In alternative embodiments, the first positioner 107a is configured to translate (e.g., advance or retract) the harvesting cannula 200. The second engagement portion 124 of the holding unit 109 is coupled to the second positioner 107b, which is configured to rotate the harvesting cannula 200 about its axis. The third engagement portion 126 of the holding unit 109 is coupled to the third positioner 107c, which is configured to translate (e.g., advance or retract) the implanting cannula 202.

In other embodiments, the second engagement portion 124 of the holding unit 109 may be coupled to both the first positioner 107a and the second positioner 107b. In such cases, the first positioner 107a is configured to translate the engagement portion 124 to thereby advance or retract the harvesting cannula 200, and the second positioner 107b is configured to rotate the engagement portion 124 to thereby turn the harvesting cannula 200 about its axis. In further embodiments, the second positioner 107b is not needed, and the cannula assembly 110 does not include the engagement portion 238. In such cases, the positioning assembly 106 is not configured to rotate the harvesting cannula 200, but to advance and retract the harvesting cannula 200 in a back and forth trusting motion. In still further embodiments, the third positioner 107c is not needed, and the third engagement portion 126 is fixedly secured to the holding unit 109. In such cases, the implanting cannula 202 may be positioned by the robotic arm 27, and the harvesting cannula 200 may be positioned relative to the implanting cannula 202 using the first positioner 107a.

When using the cannula assembly 110 to harvest a follicular unit, the cannula assembly 110 is first coupled to the positioning assembly 106. Such may be accomplished manually by snapping the cannula assembly 110 onto the positioning assembly 106. Alternatively, the cannula assembly 110 may be held upright by a stand (not shown). In such cases, the robotic arm 27 may be used to move the positioning assembly 106 to "grab" the cannula assembly 110 from the stand. The camera(s) 28 may be used to provide information regarding a position of the cannula assembly 110 to the processor 120, which controls the robotic arm 27 based on the information, thereby placing the positioning assembly 106 in engagement position relative to the cannula assembly 110.

Next, a treatment plan is input into the computer 120. In some embodiments, the treatment plan is a prescribed plan designed to transplant hair follicular units from a first region (harvest region) to a target region (implant region). In such cases, the treatment plan may include one or more parameters, such as a number of hair follicular units to be removed/implanted, location of harvest region, location of implant region, a degree of randomness associated with targeted implant locations, spacing between adjacent targeted implant locations, depth of follicle, depth of implant, patient identification, geometric profile of harvest region, geometric profile of implant region, marker location(s), and density of targeted implant locations. Various techniques may be used to input the treatment plan into the computer 120. In the illustrated embodiments, the treatment plan may be input using a user interface that includes a monitor 122 and a keyboard 124. Alternatively, the treatment plan may be input using a storage device, such as a diskette or a compact disk. In other embodiments, the treatment plan may be downloaded from a remote server. In further embodiments, the treatment plan may be input using a combination of the above techniques. For example, some parameters may be input into the computer 120 using a diskette, while other parameters may be input using the user interface. In some embodiments, one or more parameters of the treatment plan may be determined in real time (e.g., during a treatment session).

After the treatment plan has been input into the computer 120, the computer 120 then registers the treatment plan with a patient. In some embodiments, such may be accomplished by using the camera(s) 28 to identify one or more markers on the patient. The marker may be a reflector that is secured to the patient, an ink mark drawn on the patient, or an anatomy of the patient. The identified marker(s) may be used to determine a position and/or orientation of a target region on the patient. In the illustrated embodiments, the treatment plan includes a position of the harvest (or donor) region. Using input from the camera(s) 28, the computer 120 identifies the location of the harvest region on the patient, and a target follicular unit in the harvest region. The computer 120 then operates the robotic arm 27 to place the distal end 214 of the harvesting cannula 200 next to the target follicular unit. In some embodiments, the harvesting cannula 200 is positioned coaxially with the target follicular unit.

Next, the harvesting cannula 200 is used to harvest the target follicular unit. In some embodiments, such may be accomplished by activating a positioner within the positioning assembly 106 to rotate the harvesting cannula 200. As the harvesting cannula 200 is rotated, the harvesting cannula 200 may be advanced distally (e.g., by activating another positioner within the positioning assembly 106, or by moving the positioning assembly 106 using the robotic arm 27). In other embodiments, the harvesting of the target follicle 302 unit may be accomplished by thrusting the harvesting cannula 200 forward and backward. While the harvesting cannula 200 is used to core out the follicular unit 302, the implanting cannula 202 is located proximally away from the distal end 214 of the harvesting cannula 200 to thereby prevent interference with the harvesting procedure. Such may be accomplished by advancing the harvesting cannula 200 distally relative to the implanting cannula 202, or alternatively, by retracting the implanting cannula 202 proximally relative to the harvesting cannula 200 (if the implanting cannula 202 can be positioned).

When the distal end 214 of the harvesting cannula 200 has been advanced within a prescribed depth, e.g., 5 millimeter, below the skin surface, the harvesting cannula 200 is then retracted and removed from the patient. The camera(s) 28 may be used to monitor the harvesting process to thereby determine how far the harvesting cannula 200 has been advanced below the skin surface 306. In some embodiments, the exterior of the harvesting cannula 200 may include marker lines to thereby allow the camera(s) 28 or a physician to "see" how much of the harvesting cannula 200 has been advanced into the patient. In some embodiments, surface friction at the interface between the follicular unit 302 and the interior surface 304 within the lumen 217 will hold the follicular unit 302 as the harvesting cannula 200 is removed from the patient, thereby harvesting the follicular unit 302.

In other embodiments, the interior surface 304 can be texturized (e.g., having one or more indents or protrusions) to thereby allow the distal end 214 to more easily hold onto the follicular unit 302 as the harvesting cannula 200 is removed from the patient. In further embodiments, a proximal end of the cannula assembly 110 may be coupled to a vacuum unit (not shown) located within the positioning assembly 106. In such cases, the vacuum unit creates suction within the lumen 217 of the harvesting cannula 200, to thereby pull the target follicular unit 302 away from its underlying tissue as the harvesting cannula 200 is removed from the patient.

After the follicular unit 302 has been harvested, the positioning assembly 106 retracts the harvesting cannula 200 proximally until the distal end 214 is proximal to the distal end 234 of the implanting cannula 202. Alternatively, if the implanting cannula 202 is positionable, the implanting cannula 202 may be advanced distally until the distal end 234 is distal to the distal end 214 of the harvesting cannula 200. Next, the computer 120 operates the robotic arm 27 to place the distal end 234 of the implanting cannula 202 adjacent to a target location within an implant region of the patient as prescribed by the treatment plan. The implanting cannula 202 is then advanced (e.g., by activating a positioner within the positioning assembly 106, or by moving the positioning assembly 106 distally towards the target location) to pierce through the skin 310 at the implant region (Fig. 1 IA). The implanting cannula 202 is advanced until the penetrated depth 312 is at least equal to the coring depth 300. In some embodiments, the camera(s) 28 and the computer 120 may be used to determine an amount of the implanting cannula 202 that has been advanced into the patient. For example, the implanting cannula 202 may include a plurality of marker lines for allowing the camera(s) 28 or a physician to "see" how much of the implanting cannula 202 has been inserted into the patient. As shown in the figure, the implanting cannula 202 creates an opening 314 below the patient's skin 314, in which the follicular unit 302 may be placed.

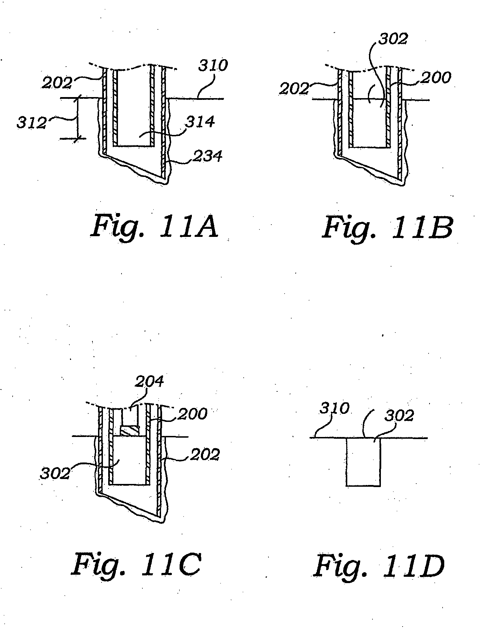

Next, the harvesting cannula 200, which contains the harvested follicular unit 302, is advanced within the lumen 236 of the implanting cannula 202, until a top surface 320 of the follicular unit 302 is at or below the skin 310 at the implant region (Fig. 1 IB). Next, the plunger 204 may be advanced distally (e.g., by using another positioner within the positioning assembly 106) until its distal end 244 engages with the follicular unit 302 located within the harvesting cannula 200 (Fig. 11C). The implanting cannula 202 and the harvesting cannula 200 are then retracted proximally relative to the plunger 204, thereby leaving the follicular unit 302 implanted at the target location in the implant region (Fig. 1 ID). In other embodiments, the cannula assembly 110 does not include the plunger 204. In such cases, a pressure generator (not shown) located within the positioning assembly 106 may be used to create a pressure within the lumen 217 of the harvesting cannula 200, thereby pushing the follicular unit 302 towards the patient as the implanting cannula 202 and the harvesting cannula 200 is retracted. Such technique will cause the follicular unit 302 to dislodge from the harvesting cannula 200 while the harvesting cannula 200 is being removed from the patient.

After the first follicular unit 302 has been implanted in the implant region, the harvesting cannula 200 is advanced distally until its distal end 214 is distal to the distal end 234 of the implanting cannula 202. The computer 120 then operates the robotic arm 27 again to place the harvesting cannula 200 next to another target follicular unit 302 to be harvested. The above described process is then repeated to harvest the next follicular unit 302, and to implant the follicular unit 302. The selection of the follicular unit 302 may be determined by the computer 120. For example, in some embodiments, based on a location and geometry of the prescribed harvest region, the computer 120 selects a follicular unit 302 only if it is within the prescribed harvest region. In some embodiments, the above process is repeated until a prescribed number of follicular units 302 have been implanted in the implant region, until a density of the implanted follicle units 302 reaches a prescribed density, or until there is no more available follicular unit 302 in the harvest region.

In some embodiments of the invention employing an automated positioning system, an attending physician or operator may still specify where a follicular unit needs to be implanted and at what angle, i.e., its relative location (or "implantation site"), orientation, and depth. For example, specification of a location, orientation, and/or depth of a follicular unit to be implanted may be carried out by a treatment planning system. Alternatively, during the implanting mode, when the camera(s) are viewing the recipient area of the scalp, the attending operator may use a user interface (e.g., a conventional computer mouse) to specify the implant location and/or position and/or orientation and/or implant depth. Alternatively, the operator can point to location on the scalp by placing a temporary fiducial, such as an ink mark or a pointer that can be visualized, identified, and measured by the image processing system. Further, orientation can be specified directly on the computer monitor as a combination of two angles, such as rotation about x-axis and a rotation about y-axis (assuming that z-axis is along the cannula), or by placing an elongated pointer on the scalp, which the image processing system can visualize and measure the angles.

In any case, the control of the robotic arm now becomes two steps. First, based on the specification of the location and orientation of the implant location, the computer processor directs the robotic arm to move the implanting cannula to a desired location and orientation. Second, the actual advancement of the implanting cannula into the skin surface takes place, either solely by actuating the mechanism, or by a combination of robotic arm movement and mechanism actuation, in which the desired implant depth is achieved. Another way of specifying the orientation of the implanted follicular unit is to have the system match to the orientation of the one or more hair follicles extending there from to the orientation of existing hair follicles in the area of implantation. The system, after positioning the implanting cannula at the implantation location, visualizes and measures the orientation of neighboring hair follicles, and uses this information to determine an appropriate orientation of the follicular unit being implanted. In the case of neighboring hair follicles having different orientations, the system may, for example, obtain a weighted average of the various orientations for determining an orientation of the follicular unit being implanted.

## Claims

1. An apparatus for harvesting and implanting hair follicular units, comprising:
an outer implanting cannula (36, 202) having an interior lumen and an open, tissue-piercing distal end (37, 234) configured for implanting a follicular unit into an implant region; and
an inner harvesting cannula (38, 200) positioned coaxially relative to the implanting cannula (36, 202), the harvesting cannula (38,200) having an open, tissue-coring distal end (40, 214) and an interior lumen (217), the interior lumen (217) is sized to allow an entire follicular unit to be encapsulated and fit therein,
wherein the harvesting and implanting cannulas are moveable relative to each other in a reciprocating relationship, **characterized in that** when penetrating a donor region to harvest hair follicular units, the distal end (37, 234) of the outer implanting cannula (36,202) is located proximal to the distal end (40, 214) of the inner harvesting cannula (38, 200).

2. The apparatus of claim 1, wherein when puncturing an implant region to form an implantation cavity, the distal end of the implanting cannula is advanced past the distal end of the harvesting cannula.

3. The apparatus of claim 1 or claim 2, wherein the apparatus is selected from the group comprising a hand-held device, a substantially automated device, and a device configured to be maneuverable by a robotic system.

4. The apparatus of claim 1 or claim 2, the apparatus further comprising a moveable arm and wherein the implanting and the harvesting cannulas are parts of a multi-part tool assembly coupled to the moveable arm.

5. The apparatus of claim 4, wherein the apparatus is a robotic apparatus and the moveable arm is a robotic arm.

6. The apparatus of any of claims 4-5, wherein one or both of the implanting and harvesting cannulas are removable from the multi-part tool assembly.

7. The apparatus of any of claims 4-6, wherein the harvesting cannula is adapted to move relative to the body surface by one or more of (1) movement of the harvesting cannula relative to the tool assembly, (2) movement of the moveable arm, or (3) movement of the harvesting cannula relative to the moveable arm.

8. The apparatus of any of claims 1-7, the apparatus further comprising one or more of a mechanical system, electromechanical system, pneumatic system, hydraulic system or magnetic system configured to effect movement of the harvesting and implanting cannulas relative to each other.

9. The apparatus of any of claims 1-7, the apparatus further comprising a motor drive assembly configured to operatively couple with the implanting and harvesting cannulas to provide automated translational movement of at least one of the implanting and harvesting cannulas relative to the other.

10. The apparatus of any of claims 1-9, wherein the apparatus is configured to provide a rotational movement of the harvesting cannula about its longitudinal axis.

11. The apparatus of any of claims 1-10, further comprising a processor configured to determine a position of the implanting and harvesting cannulas based at least in part on image data received from one or more cameras.

12. The apparatus of any of claims 1-10, the apparatus further comprising a force sensor and a control circuitry, and wherein the control circuitry is configured to stop harvesting or implanting process if a force sensed by the force sensor exceeds a prescribed limit.

13. The apparatus of any of claims 1-12, further comprising a source of pressure in communication with the lumen of the harvesting cannula.

14. The apparatus of any of claims 1-13, wherein at least a portion of an interior wall of the harvesting cannula is texturized.

15. The apparatus of any of claims 1-14, the apparatus further comprising an obturator and wherein the obturator is co-axial and movable relative to one or both of the harvesting and the implanting cannulas.

## Patentansprüche

1. Gerät zur Gewinnung und Implantation von haarfollikulären Einheiten, umfassend:
eine äußere Implantationskanüle (36, 202) mit einem Innenlumen und einem offenen, gewebedurchstechenden distalen Ende (37, 234), konfiguriert zur Implantation einer follikulären Einheit in eine Implantationsregion; und
eine innere Gewinnungskanüle (38, 200), die koaxial zu der Implantationskanüle (36, 202) angeordnet ist, wobei die Gewinnungskanüle (38, 200) ein offenes, gewebedurchstechendes distales Ende (40, 214) und ein Innenlumen (217) hat, wobei die Größe des Innenlumens (217) so ist, dass sie es erlaubt, dass eine ganze follikuläre Einheit darin eingekapselt werden kann und hineinpasst,
wobei die Gewinnungs- und Implantationskanülen zueinander in einer Pendelbeziehung beweglich sind, **dadurch gekennzeichnet, dass** beim Eindringen in eine Donorregion zur Gewinnung haarfollikulärer Einheiten das distale Ende (37, 234) der äußeren Implantationskanüle (36, 202) proximal zu dem distalen Ende (40, 214) der inneren Gewinnungskanüle (38, 200) angeordnet ist.

2. Gerät nach Anspruch 1, wobei beim Punktieren einer Implantationsregion zum Bilden eines Implantationshohlraums das distale Ende der Implantationskanüle vorbei am distalen Ende der Gewinnungskanüle vorwärts bewegt wird.

3. Gerät nach Anspruch 1 oder Anspruch 2, wobei das Gerät ausgewählt ist aus der Gruppe umfassend eine in der Hand gehaltene Vorrichtung, eine im Wesentlichen automatisierte Vorrichtung und eine Vorrichtung, konfiguriert um von einem Robotersystem manövriert zu werden.

4. Gerät nach Anspruch 1 oder Anspruch 2, wobei das Gerät ferner einen beweglichen Arm umfasst, und wobei die Implantations- und Gewinnungskanülen Teile einer mehrteiligen, an den beweglichen Arm gekoppelten Werkzeuganordnung sind.

5. Gerät nach Anspruch 4, wobei das Gerät ein Robotergerät ist, und der bewegliche Arm ein Roboterarm ist.

6. Gerät nach einem der Ansprüche 4-5, wobei eine oder beide der Implantations- und Gewinnungskanülen von der mehrteiligen Werkzeuganordnung entfernt werden können.

7. Gerät nach einem der Ansprüche 4-6, wobei die Gewinnungskanüle dazu angepasst ist, sich zu der Körperoberfläche zu bewegen durch eine oder mehr von (1) Bewegung der Gewinnungskanüle relativ zu der Werkzeuganordnung, (2) Bewegung des beweglichen Arms, oder (3) Bewegung der Gewinnungskanüle relativ zu dem beweglichen Arm.

8. Gerät nach einem der Ansprüche 1-7, wobei das Gerät ferner eines oder mehrere eines mechanischen Systems, eines elektromechanischen Systems, eines pneumatischen Systems, eines hydraulischen Systems oder eines magnetischen Systems umfasst, konfiguriert zur Ausführung einer Bewegung der Gewinnungs- und Implantationskanülen relativ zueinander.

9. Gerät nach einem der Ansprüche 1-7, wobei das Gerät ferner eine Motorantriebsanordnung umfasst, konfiguriert zum funktionsfähigen Koppeln mit den Implantations- und Gewinnungskanülen, um eine automatische translatorische Bewegung von mindestens einer der Implantations- und Gewinnungskanülen relativ zueinander bereitzustellen.

10. Gerät nach einem der Ansprüche 1-9, wobei das Gerät konfiguriert ist, um eine Drehbewegung der Gewinnungskanüle um ihre Längsachse bereitzustellen.

11. Gerät nach einem der Ansprüche 1-10, ferner umfassend einen Prozessor, konfiguriert zur Bestimmung einer Position der Implantations- und Gewinnungskanülen basierend mindestens teilweise auf Bilddaten, empfangen von einer oder mehreren Kameras.

12. Gerät nach einem der Ansprüche 1-10, wobei das Gerät ferner einen Kraftsensor und einen Steuerkreis umfasst, und wobei der Steuerkreis konfiguriert ist, um den Gewinnungs- und Implantationsprozess zu stoppen, wenn eine vom Kraftsensor wahrgenommene Kraft eine vorgeschriebene Grenze überschreitet.

13. Gerät nach einem der Ansprüche 1-12, ferner umfassend eine Druckquelle, die in Kommunikation mit dem Lumen der Gewinnungskanüle ist.

14. Gerät nach einem der Ansprüche 1-13, wobei mindestens ein Teil einer Innenwand der Gewinnungskanüle texturiert ist.

15. Gerät nach einem der Ansprüche 1-14, wobei das Gerät ferner einen Obturator umfasst, und wobei der Obturator koaxial und beweglich relativ zu einer oder beiden der Gewinnungs- und Implantationskanülen ist.

## Revendications

1. Dispositif de récolte et d'implantation d'unités folliculaires de cheveux, comprenant :
une canule d'implantation (36, 202) extérieure comportant une lumière intérieure et une extrémité distale (37, 234) de percement de tissu, ouverte configurée pour implanter une unité folliculaire dans une région d'implantation ; et
une canule de récolte (38, 200) intérieure positionnée coaxialement relativement à la canule d'implantation (36, 202), la canule de récolte (38, 200) comportant une extrémité distale (40, 214) de carottage de tissu, ouverte et une lumière intérieure (217), la lumière intérieure (217) est dimensionnée pour permettre à une unité folliculaire entière d'être encapsulée et installée à l'intérieur,
dans lequel les canules de récolte et d'implantation sont mobiles relativement l'une à l'autre dans une relation de va-et-vient, **caractérisé en ce que** lors de la pénétration dans une région de donneur pour récolter des unités folliculaires, l'extrémité distale (37, 234) de la canule d'implantation (36, 202) extérieure est située proche de l'extrémité distale (40, 214) de la canule de récolte (38, 200) intérieure.

2. Dispositif selon la revendication 1, dans lequel lors de la perforation d'une région d'implantation pour former une cavité d'implantation, l'extrémité distale de la canule d'implantation est avancée au-delà de l'extrémité distale de la canule de récolte.

3. Dispositif selon la revendication 1 ou la revendication 2, dans lequel le dispositif est sélectionné dans le groupe comprenant un dispositif tenu à la main, un dispositif sensiblement automatisé, et un dispositif configuré pour pouvoir être manoeuvré par un système robotique.

4. Dispositif selon la revendication 1 ou la revendication 2, le dispositif comprenant en outre un bras mobile et dans lequel les canules d'implantation et de récolte sont des parties d'un ensemble d'outil à parties multiples couplé au bras mobile.

5. Dispositif selon la revendication 4, dans lequel le dispositif est un dispositif robotique et le bras mobile est un bras robotique.

6. Dispositif selon l'une quelconque des revendications 4-5, dans lequel une ou les deux des canules d'implantation et de récolte peuvent être retirées de l'ensemble d'outil à parties multiples.

7. Dispositif selon l'une quelconque des revendications 4-6, dans lequel la canule de récolte est adaptée pour se déplacer relativement à la surface de corps par un ou plusieurs parmi (1) un déplacement de la canule de récolte relativement à l'ensemble d'outil, (2) un déplacement du bras mobile, ou (3) un déplacement de la canule de récolte relativement au bras mobile.

8. Dispositif selon l'une quelconque des revendications 1-7, le dispositif comprenant en outre l'un ou plusieurs d'un système mécanique, un système électromécanique, un système pneumatique, un système hydraulique ou un système magnétique configurés pour effectuer le déplacement des canules d'implantation et de récolte relativement l'une à l'autre.

9. Dispositif selon l'une quelconque des revendications 1-7, le dispositif comprenant en outre un ensemble d'entraînement motorisé configuré pour être couplé fonctionnellement avec les canules d'implantation et de récolte pour permettre un déplacement en translation automatisé d'au moins l'une des canules d'implantation et de récolte relativement à l'autre.

10. Dispositif selon l'une quelconque des revendications 1-9, dans lequel le dispositif est configuré pour permettre un déplacement en rotation de la canule de récolte autour de son axe longitudinal.

11. Dispositif selon l'une quelconque des revendications 1-10, comprenant en outre un processeur configuré pour déterminer une position des canules d'implantation et de récolte en fonction au moins en partie de données d'images reçues d'une ou plusieurs caméras.

12. Dispositif selon l'une quelconque des revendications 1-10, le dispositif comprenant en outre un capteur de force et un circuit de commande, et dans lequel le circuit de commande est configuré pour stopper le processus de récolte et d'implantation si une force détectée par le capteur de force dépasse une limite prescrite.

13. Dispositif selon l'une quelconque des revendications 1-12, comprenant en outre une source de pression en communication avec la lumière de la canule de récolte.

14. Dispositif selon l'une quelconque des revendications 1-13, dans lequel au moins une partie d'une paroi intérieure de la canule de récolte est texturisée.

15. Dispositif selon l'une quelconque des revendications 1-14, le dispositif comprenant en outre un obturateur et dans lequel l'obturateur est coaxial et mobile relativement à l'une ou les deux des canules d'implantation et de récolte.
